# EUROPEAN PATENT APPLICATION

(11) **EP 4 230 741 A1**
(43) Date of publication of application: **23.08.2023**
(21) Application number: 21880151.2
(22) Date of filing: 13.10.2021
(51) Int. Cl.: C12N 15/50, A61K 39/215, A61P 31/12, A61P 31/14, A61P 37/04, C07K 14/115, C07K 14/165, C12N 7/01, C12N 7/04, C12N 7/08, C12N 9/16, C12N 15/01, C12N 15/45, C12N 15/55, C12Q 1/6869

(54) **BETA CORONAVIRUS COLD ACCLIMATIZED STRAIN AND VACCINE**

(30) Priority: 14.10.2020 JP 2020173494; 28.10.2020 JP 2020180524; 18.12.2020 JP 2020210564; 05.02.2021 JP 2021017633; 25.03.2021 JP 2021051107
(71) Applicant: The Research Foundation for Microbial Diseases of Osaka University, Suita-shi, Osaka 565-0871 (JP)
(72) Inventor: OKAMURA, Shinya, Suita-shi, Osaka 565-0871 (JP); KASHIWABARA, Akiho, Suita-shi, Osaka 565-0871 (JP); EBINA, Hirotaka, Suita-shi, Osaka 565-0871 (JP)
(74) Representative: Müller-Boré & Partner Patentanwälte PartG mbB
(86) International application number: PCT/JP2021/037902
(87) International publication number: WO 2022/080413

(57) **Abstract**

Strains that is effective as the active component of a vaccine against the betacoronavirus is provided. A SARS-CoV-2 containing structural protein(s) and/or non-structural protein(s) having the following mutation(s): the amino acid residue mutations in NSP3, corresponding to V at position 404, L at position 445, K at position 1792 and/or D at position 1832 in SEQ ID No. 1; the amino acid residue mutations in NSP14, corresponding to G at position 248, G at position 416, and/or A at position 504 in SEQ ID No. 2; the amino acid residue mutation in NSP16, corresponding to V at position 67 in SEQ ID No. 3; the amino acid residue mutations in the spike, corresponding to L at position 54, T at position 739 and/or A at position 879 in SEQ ID No. 4; the amino acid residue mutation in the envelope, corresponding to L at position 28 in SEQ ID No. 5; and/or, the amino acid residue mutation in the nucleocapsid, corresponding to S at position 2 in SEQ ID No. 6;

## Description

### TECHNICAL FIELD

The present invention relates to temperature-sensitive (cold-adapted) strains of betacoronavirus and a vaccine using the same.

### BACKGROUND ART

The infection with the novel coronavirus (SARS-CoV-2) as a pathogen that occurred in Wuhan, China in 2019 (COVID-19) has caused a pandemic worldwide and is a major social problem. For this reason, vaccine development for SARS-CoV-2 is progressing rapidly around the world. The only vaccine that has been approved as of October 2020 is Sputnik V that has been approved in Russia (Non-Patent Document 1).

### PRIOR ART DOCUMENT

### NON-PATENT DOCUMENT

Non-Patent Document 1: THE LANCET, VOLUME 396, ISSUE 10255, P887-897, SEPTEMBER 26, 2020

### SUMMARY OF THE INVENTION

### PROBLEMS TO BE SOLVED BY THE INVENTION

However, Sputnik V is still in the stage of conducting clinical trials, and there are raised questions about safety and efficacy. In addition, even if vaccination is started, there is still a risk that the effect is small or heavy side effects are found, and it is not clear whether the vaccination is a decisive blow to prevent the spread of an infection. Thus, additional options for vaccines against SARS-CoV-2 are desired.

Therefore, an object of the present invention is to provide at least a strain that is effective as an active ingredient of a vaccine against SARS-CoV-2 In addition, since betacoronaviruses such as SARS-CoV-2 include viruses that may be present other than SARS-CoV-2 it is an object of the present invention to provide a strain that is effective as an active ingredient of a vaccine against betacoronaviruses in general.

### MEANS FOR SOLVING THE PROBLEM

As a result of intensive studies, the present inventors have found that growth of the SARS-CoV-2 strains having specific mutation(s) at human body temperature (so-called lower respiratory tract temperature) is decreased, and have found that the specific mutation(s) can cause a decrease in growth of betacoronaviruses in general at human body temperature (so-called lower respiratory tract temperature). The present invention has been completed by further conducting studies based on these findings. Incidentally, in the present invention, the term "cold adaptation" is used in the meaning of acquiring a property having a growth capability specific to a low temperature (i.e., human upper respiratory tract temperature), and in its actual state, the term is used in the meaning of "temperature sensitization" since the growth capability specific to a low temperature is exhibited by acquiring a property in which a growth capability at a high temperature (i.e., human lower respiratory tract temperature) is limited. In addition, a "temperature-sensitized" strain is referred to as a "temperature-sensitive strain". In other words, the present invention provides inventions of the following embodiments.

Item 1. A betacoronavirus temperature-sensitive (cold-adapted) strain containing structural protein(s) and/or non-structural protein(s) having at least any one of the following mutations of (a) to (m):
(a) a mutation of an amino acid residue corresponding to valine at position 404 of an amino acid sequence set forth in SEQ ID NO: 1 in NSP3,
(b) a mutation of an amino acid residue corresponding to leucine at position 445 of an amino acid sequence set forth in SEQ ID NO: 1 in NSP3,
(c) a mutation of an amino acid residue corresponding to lysine at position 1792 of an amino acid sequence set forth in SEQ ID NO: 1 in NSP3,
(d) a mutation of an amino acid residue corresponding to aspartic acid at position 1832 of an amino acid sequence set forth in SEQ ID NO: 1 in NSP3,
(e) a mutation of an amino acid residue corresponding to glycine at position 248 of an amino acid sequence set forth in SEQ ID NO: 2 in NSP14,
(f) a mutation of an amino acid residue corresponding to glycine at position 416 of an amino acid sequence set forth in SEQ ID NO: 2 in NSP14,
(g) a mutation of an amino acid residue corresponding to alanine at position 504 of an amino acid sequence set forth in SEQ ID NO: 2 in NSP14,
(h) a mutation of an amino acid residue corresponding to valine at position 67 of an amino acid sequence set forth in SEQ ID NO: 3 in NSP16,
(i) a mutation of an amino acid residue corresponding to leucine at position 54 of an amino acid sequence set forth in SEQ ID NO: 4 in a spike,
(j) a mutation of an amino acid residue corresponding to threonine at position 739 of an amino acid sequence set forth in SEQ ID NO: 4 in a spike,
(k) a mutation of an amino acid residue corresponding to alanine at position 879 of an amino acid sequence set forth in SEQ ID NO: 4 in a spike,
(l) a mutation of an amino acid residue corresponding to leucine at position 28 of an amino acid sequence set forth in SEQ ID NO: 5 in an envelope, and
(m) a mutation of an amino acid residue corresponding to serine at position 2 of an amino acid sequence set forth in SEQ ID NO: 6 in a nucleocapsid.

Examples of the invention of the above item 1 include the following inventions.

A betacoronavirus temperature-sensitive (cold-adapted) strain containing structural protein(s) and/or non-structural protein(s) consisting of at least any one of the following polypeptides (1-1) to (I-6), (II), and (III):
(I-1) a polypeptide (NSP3) consisting of an amino acid sequence having mutation(s) of at least any one of a mutation of valine at position 404 (a'), a mutation of leucine at position 445 (b'), a mutation of lysine at position 1792 (c'), and a mutation of aspartic acid at position 1832 (d') in an amino acid sequence set forth in SEQ ID NO: 1,
(I-2) a polypeptide (NSP14) consisting of an amino acid sequence having mutation(s) of at least any one of a mutation of glycine at position 248 (e'), a mutation of glycine at position 416 (f), and a mutation of alanine at position 504 (g') in an amino acid sequence set forth in SEQ ID NO: 2,
(I-3) a polypeptide (NSP16) consisting of an amino acid sequence having a mutation of valine at position 67 (h') in an amino acid sequence set forth in SEQ ID NO: 3,
(I-4) a polypeptide (spike) consisting of an amino acid sequence having mutation(s) of at least any one of a mutation of leucine at position 54 (i'), a mutation of threonine at position 739 (j'), and a mutation of alanine at position 879 (k') in an amino acid sequence set forth in SEQ ID NO: 4,
(I-5) a polypeptide (envelope) consisting of an amino acid sequence having a mutation of leucine at position 28 (l') in an amino acid sequence set forth in SEQ ID NO: 5, and
(I-6) a polypeptide (nucleocapsid) consisting of an amino acid sequence having a mutation of serine at position 2 (m') in an amino acid sequence set forth in SEQ ID NO: 6;
(II) a polypeptide wherein in the amino acid sequence of the polypeptides (1-1) to (I-6), one or more amino acid residues other than the amino acid residues related to the mutations (a') to (m') are substituted, added, inserted, or deleted, and the polypeptide constituting a betacoronavirus that has acquired a temperature-sensitive (cold adaptation) capability; and
(III) a polypeptide having a sequence identity of 50% or more in the amino acid sequence of the polypeptides (I-1) to (I-6) excluding the amino acid residues related to the mutations (a') to (m'), and constituting a betacoronavirus that has acquired a temperature-sensitive (cold adaptation) capability.

Item 2. The virus temperature-sensitive (cold-adapted) strain according to item 1, wherein the betacoronavirus is SARS-CoV-2.

Item 3. The virus temperature-sensitive (cold-adapted) strain according to item 1 or 2, wherein a growth capability at a human lower respiratory tract temperature is decreased as compared with a growth capability of a betacoronavirus containing structural protein(s) and non-structural protein(s) not having any of the mutations of (a) to (m).

Item 4. The virus temperature-sensitive (cold-adapted) strain according to item 3, wherein the human lower respiratory tract temperature is 36 to 38°C.

Item 5. The virus temperature-sensitive (cold-adapted) strain according to any one of items 1 to 4, wherein the mutation of (a) is a substitution with alanine, the mutation of (b) is a substitution with phenylalanine, the mutation of (c) is a substitution with arginine, the mutation of (d) is a substitution with asparagine, the mutation of (e) is a substitution with valine, the mutation of (f) is a substitution with serine, the mutation of (g) is a substitution with valine, the mutation of (h) is a substitution with isoleucine, the mutation of (i) is a substitution with tryptophan, and/or the mutation of (j) is a substitution with lysine, the mutation of (k) is a substitution with valine, the mutation of (1) is a substitution with proline, and/or the mutation of (m) is a substitution with phenylalanine.

Item 6. The virus temperature-sensitive (cold-adapted) strain according to any one of items 1 to 5, containing:
the NSP3 having the mutation of (a), the mutation of (b), the mutation of (c), and/or the mutation of (d) in the amino acid sequence set forth in SEQ ID NO: 1;
the NSP14 having the mutation of (e), the mutation of (f), and/or the mutation of (g) in the amino acid sequence set forth in SEQ ID NO: 2;
the NSP16 having the mutation of (h) in the amino acid sequence set forth in SEQ ID NO: 3;
the spike having the mutation of (i), the mutation of (j), and/or the mutation of (k) in the amino acid sequence set forth in SEQ ID NO: 4;
the envelope having the mutation of (1) in the amino acid sequence set forth in SEQ ID NO: 5; and/or
the nucleocapsid having the mutation of (m) in the amino acid sequence set forth in SEQ ID NO: 6.

Item 7. The virus temperature-sensitive (cold-adapted) strain according to any one of items 1 to 6, having the mutation of (e), the mutation of (f), the mutation of (g), the mutation of (k), the mutation of (1), and the mutation of (m).

Item 8. The virus temperature-sensitive (cold-adapted) strain according to any one of items 1 to 6, having the mutation of (a), the mutation of (d), the mutation of (h), and the mutation of (j).

Item 9. The virus temperature-sensitive (cold-adapted) strain according to any one of items 1 to 6, having the mutation of (b) and the mutation of (c).

Item 10. The virus temperature-sensitive (cold-adapted) strain according to any one of items 1 to 6, having the mutation of (b), the mutation of (c), and the mutation of (i).

Item 11. The virus temperature-sensitive (cold-adapted) strain according to any one of items 8 to 10, having a deletion of an amino acid sequence encoded by a base sequence set forth in SEQ ID NO: 7.

Item 12. A live attenuated vaccine containing the virus temperature-sensitive (cold-adapted) strain according to any one of items 1 to 11.

Item 13. The live attenuated vaccine according to claim 12, which is administered nasally.

Item 14. The live attenuated vaccine according to item 12, which is administered intramuscularly, subcutaneously, or intradermally.

Item 15. A betacoronavirus gene vaccine containing a gene encoding structural protein(s) and/or non-structural protein(s) having at least any one of the following mutations of (a) to (m):
(a) a mutation of an amino acid residue corresponding to valine at position 404 of an amino acid sequence set forth in SEQ ID NO: 1 in NSP3,
(b) a mutation of an amino acid residue corresponding to leucine at position 445 of an amino acid sequence set forth in SEQ ID NO: 1 in NSP3,
(c) a mutation of an amino acid residue corresponding to lysine at position 1792 of an amino acid sequence set forth in SEQ ID NO: 1 in NSP3,
(d) a mutation of an amino acid residue corresponding to aspartic acid at position 1832 of an amino acid sequence set forth in SEQ ID NO: 1 in NSP3,
(e) a mutation of an amino acid residue corresponding to glycine at position 248 of an amino acid sequence set forth in SEQ ID NO: 2 in NSP14,
(f) a mutation of an amino acid residue corresponding to glycine at position 416 of an amino acid sequence set forth in SEQ ID NO: 2 in NSP14,
(g) a mutation of an amino acid residue corresponding to alanine at position 504 of an amino acid sequence set forth in SEQ ID NO: 2 in NSP14,
(h) a mutation of an amino acid residue corresponding to valine at position 67 of an amino acid sequence set forth in SEQ ID NO: 3 in NSP16,
(i) a mutation of an amino acid residue corresponding to leucine at position 54 of an amino acid sequence set forth in SEQ ID NO: 4 in a spike,
(j) a mutation of an amino acid residue corresponding to threonine at position 739 of an amino acid sequence set forth in SEQ ID NO: 4 in a spike,
(k) a mutation of an amino acid residue corresponding to alanine at position 879 of an amino acid sequence set forth in SEQ ID NO: 4 in a spike,
(l) a mutation of an amino acid residue corresponding to leucine at position 28 of an amino acid sequence set forth in SEQ ID NO: 5 in an envelope, and
(m) a mutation of an amino acid residue corresponding to serine at position 2 of an amino acid sequence set forth in SEQ ID NO: 6 in a nucleocapsid.

Item 16. The gene vaccine according to item 15, which is administered nasally, intramuscularly, subcutaneously, or intradermally.

### ADVANTAGES OF THE INVENTION

According to the present invention, there is provided a strain that is effective as an active ingredient of a vaccine against a betacoronavirus.

### BRIEF DESCRIPTION OF THE DRAWINGS

Fig. 1 shows a method for temperature sensitization (cold adaptation) of SARS-CoV-2.
Fig. 2 shows results of evaluation of temperature sensitivity (cold-adaptation) of SARS-CoV-2 by observation of CPEs
Fig. 3 shows results of mutation analysis of each virus strain.
Fig. 4A shows results of growth dynamics of a temperature-sensitive strain (cold-adapted strain) (A50-18).
Fig. 4B shows results of growth dynamics of a temperature-sensitive strain (cold-adapted strain) (A50-18).
Fig. 5 shows weight changes of SARS-CoV-2-infected hamsters.
Fig. 6 shows weight changes of SARS-CoV-2-infected hamsters.
Fig. 7 shows viral loads in the lungs or nasal wash.
Fig. 8 shows images of the lungs of SARS-CoV-2-infected hamsters.
Fig. 9 shows results of histological analysis of the lungs of SARS-CoV-2-infected hamsters.
Fig. 10 shows histological analysis (HE staining and IHC staining) of the lungs of SARS-CoV-2-infected hamsters.
Fig. 11 shows weight changes of SARS-CoV-2-re-infected hamsters.
Fig. 12 shows weight changes of hamsters after SARS-CoV-2 infection.
Fig. 13 shows neutralizing antibody titers in serum of hamsters recovered after SARS-CoV-2 infection.
Fig. 14 shows methods for temperature sensitization (cold adaptation) of SARS-CoV-2 (G to L50 series).
Fig. 15 shows results of evaluation of temperature sensitivity (cold-adaptation) of SARS-CoV-2 strains by observation of CPEs.
Fig. 16 shows results of mutation analysis of additional isolates.
Fig. 17 shows a deletion of a base sequence found in relation to temperature-sensitive strains (cold-adapted strains) (H50-11, L50-33, and L50-40).
Fig. 18 shows a schematic overview of the deletion of the base sequence shown in Fig. 17 and a deletion of an amino acid sequence encoded thereby.
Fig. 19 shows results of growth dynamics of temperature-sensitive strains (cold-adapted strains) (H50-11, L50-33, and L50-40).
Fig. 20 shows weight changes of SARS-CoV-2-infected hamsters.
Fig. 21 shows lung weight of SARS-CoV-2-infected hamsters.
Fig. 22 shows viral loads in the lungs or nasal wash
Fig. 23 shows weight changes of SARS-CoV-2-re-infected hamsters.
Fig. 24 shows neutralizing antibody titers in serum of hamsters after SARS-CoV-2 infection.
Fig. 25 shows evaluation of neutralizing activity of a temperature-sensitive strain (cold-adapted strain) against a SARS-CoV-2 mutant strain.
Fig. 26 shows a comparison of immunogenicity depending on administration routes of a temperature-sensitive strain (cold-adapted strain).
Fig. 27 shows a comparison of immunogenicity depending on doses of a temperature-sensitive strain (cold-adapted strain).
Fig. 28 shows evaluation of neutralizing activity of a temperature-sensitive strain (cold-adapted strain) against SARS-CoV-2 variant strains.
Fig. 29 shows evaluation of neutralizing activity of a temperature-sensitive strain (cold-adapted strain) against SARS-CoV-2 variant strain.

### EMBODIMENTS OF THE INVENTION

### 1. Betacoronavirus temperature-sensitive strain (cold-adapted strain)

A betacoronavirus temperature-sensitive strain (cold-adapted strain) of the present invention is a betacoronavirus containing structural protein(s) and/or non-structural protein(s) having predetermined mutation(s), and is characterized by being temperature- sensitive.

The coronavirus is morphologically spherical with a diameter of about 100 to 200 nm and has protrusions on the surface. The coronavirus is virologically classified into Nidovirales, Coronavirinae, Coronaviridae. In the envelope of the lipid bilayer membrane, there is a genome of positive-stranded single-stranded RNA wound around a nucleocapsid protein (also referred to as a nucleocapsid), and a spike protein (hereinafter also referred to as a "spike"), an envelope protein (hereinafter also referred to as an "envelope"), and a membrane protein are arranged on the surface of the envelope. The size of the viral genome is about 30 kb, the longest among RNA viruses.

Coronaviruses are classified into groups of alpha, beta, gamma, and delta from genetic characteristics. As coronaviruses infecting humans, four types of human coronaviruses 229E, OC43, NL63, and HKU-1 as causative viruses of cold, and severe acute respiratory syndrome (SARS) coronavirus that occurred in 2002 and Middle East respiratory syndrome (MERS) coronavirus that occurred in 2012, which cause serious pneumonia, are known. Human coronaviruses 229E and NL63 are classified into Alphacoronavirus genus, and human coronaviruses OC43, HKU-1, SARS coronavirus, and MERS coronavirus are classified into Betacoronavirus genus.

SARS-CoV-2 classified as SARS coronavirus has been isolated and identified as a causative virus of the novel coronavirus infection that occurred in Wuhan in 2019. SARS-CoV-2 has been mutated repeatedly from the early Wuhan strain, and variant strains such as a strain detected in the United Kingdom, a strain detected in South Africa, and a strain detected in India have been found. There is also a possibility that there is a variant strain that has not yet been detected or a new variant strain will occur in the future. In the present invention, viruses included in the Betacoronavirus genus are not limited to the above SARS-CoV-2 strains, and include all other betacoronaviruses (other SARS-CoV-2 variant strains that will be newly detected in the future and betacoronaviruses other than SARS-CoV-2).

The predetermined mutations possessed by the betacoronavirus temperature-sensitive strain (cold-adapted strain) of the present invention is described based on Table 1 below. "Mutation" shown in Table 1 refers to a mutation for acquiring a temperature-sensitive (cold adaptation) capability. In Table 1, the items shown in "Amino acid after mutation" and "In the case of mutant of SARS-CoV-2 of NC#045512 (NCBI)" are examples.

**[Table 1]**

| Mutation sign | Polypeptide | Amino acid before mutation | Amino acid after mutation | In the case of mutant of SARS-CoV-2 of NC_045512 (NCBI) | | |
|---|---|---|---|---|---|---|
| | | | | SEQ ID NO | Mutation sign in the left SEQ ID NO | Mutation position in the left SEQ ID NO |
| (a) | NSP3 | Valine (V) | Alanine (A) | 1 | (a') | 404 |
| (b) | NSP3 | Leucine (L) | Phenylalanine (F) | 1 | (b') | 445 |
| (c) | NSP3 | Lysine (K) | Arginine (R) | 1 | (c') | 1792 |
| (d) | NSP3 | Aspartic acid (D) | Asparagine (N) | 1 | (d') | 1832 |
| (e) | NSP14 | Glycine (G) | Valine (V) | 2 | (e') | 248 |
| (f) | NSP14 | Glycine (G) | Serine (S) | 2 | (f') | 416 |
| (g) | NSP14 | Alanine (A) | Valine (V) | 2 | (g') | 504 |
| (h) | NSP16 | Valine (V) | Isoleucine (I) | 3 | (h') | 67 |
| (i) | Spike | Leucine (L) | Tryptophan (W) | 4 | (i') | 54 |
| (j) | Spike | Threonine (T) | Lysine (K) | 4 | (j') | 739 |
| (k) | Spike | Alanine (A) | Valine (V) | 4 | (k') | 879 |
| (l) | Envelope | Leucine (L) | Proline (P) | 5 | (l') | 28 |
| (m) | Nucleocapsid | Serine (S) | Phenylalanine (F) | 6 | (m') | 2 |

The predetermined mutations possessed by the betacoronavirus temperature-sensitive strain (cold-adapted strain) of the present invention is at least any one of the following mutations of (a) to (m).
(a) a mutation of an amino acid residue corresponding to valine at position 404 of an amino acid sequence set forth in SEQ ID NO: 1 in NSP3,
(b) a mutation of an amino acid residue corresponding to leucine at position 445 of an amino acid sequence set forth in SEQ ID NO: 1 in NSP3,
(c) a mutation of an amino acid residue corresponding to lysine at position 1792 of an amino acid sequence set forth in SEQ ID NO: 1 in NSP3,
(d) a mutation of an amino acid residue corresponding to aspartic acid at position 1832 of an amino acid sequence set forth in SEQ ID NO: 1 in NSP3,
(e) a mutation of an amino acid residue corresponding to glycine at position 248 of an amino acid sequence set forth in SEQ ID NO: 2 in NSP14,
(f) a mutation of an amino acid residue corresponding to glycine at position 416 of an amino acid sequence set forth in SEQ ID NO: 2 in NSP14, and
(g) a mutation of an amino acid residue corresponding to alanine at position 504 of an amino acid sequence set forth in SEQ ID NO: 2 in NSP14,
(h) a mutation of an amino acid residue corresponding to valine at position 67 of an amino acid sequence set forth in SEQ ID NO: 3 in NSP16,
(i) a mutation of an amino acid residue corresponding to leucine at position 54 of an amino acid sequence set forth in SEQ ID NO: 4 in a spike,
(j) a mutation of an amino acid residue corresponding to threonine at position 739 of an amino acid sequence set forth in SEQ ID NO: 4 in a spike,
(k) a mutation of an amino acid residue corresponding to alanine at position 879 of an amino acid sequence set forth in SEQ ID NO: 4 in a spike,
(l) a mutation of an amino acid residue corresponding to leucine at position 28 of an amino acid sequence set forth in SEQ ID NO: 5 in an envelope, and
(m) a mutation of an amino acid residue corresponding to serine at position 2 of an amino acid sequence set forth in SEQ ID NO: 6 in a nucleocapsid.

SEQ ID NO: 1 is an amino acid sequence of NSP3 in SARS-CoV-2 of NC_045512 (NCBI); SEQ ID NO: 2 is an amino acid sequence of NSP14 in SARS-CoV-2 of NC_045512 (NCBI); SEQ ID NO: 3 is an amino acid sequence of NSP16 in SARS-CoV-2 of NC_045512 (NCBI); SEQ ID NO: 4 is an amino acid sequence of a spike in SARS-CoV-2 of NC_045512 (NCBI); SEQ ID NO: 5 is an amino acid sequence of an envelope in SARS-CoV-2 of NC_045512 (NCBI); and SEQ ID NO: 6 is an amino acid sequence of a nucleocapsid in SARS-CoV-2 of NC_045512 (NCBI).

"Corresponding" means that there is(are) mutation(s) at the above predetermined positions in the amino acid sequences of SEQ ID NOs: 1 to 6 when the betacoronavirus temperature-sensitive strain (cold-adapted strain) of the present invention is a mutant strain of SARS-CoV-2 of NC_045512 (NCBI), and that there are mutation(s) at positions corresponding to the above predetermined positions in the amino acid sequences corresponding to SEQ ID NOs: 1 to 6 of the polypeptides possessed by another betacoronavirus variant when the betacoronavirus temperature-sensitive strain (cold-adapted strain) of the present invention is another betacoronavirus mutant strain other than the above variant. The corresponding position can be identified by aligning amino acid sequences for proteins of SEQ ID NOs: 1 to 6 of SARS-CoV-2 of NC_045512 (NCBI) and proteins of another betacoronavirus mutant strain corresponding to the proteins of SEQ ID NOs: 1 to 6.

The virus temperature-sensitive strain (cold-adapted strain) of the present invention is not limited to a mutant strain of the SARS-CoV-2 listed in NC_045512 (NCBI) as long as amino acid residues corresponding to the above predetermined positions in the amino acid sequence of SEQ ID NOs: 1 to 6 is mutated, and includes other betacoronavirus mutant strains (i.e., other any variants of SARS-CoV-2 and mutant strains of viruses other than SARS-CoV-2 included in the Betacoronavirus genus). The specific mutant strain of the SARS-CoV-2 listed in NC_045512 (NCBI) are defined as mutant strain in which at least any one of amino acid residues at the above predetermined positions in the amino acid sequences represented by SEQ ID NOs: 1 to 6 in the specific SARS-CoV-2 is mutated, and the other betacoronavirus mutant strains refer to both any other mutant strains of SARS-CoV-2 (i.e., mutant strains in which amino acid residues corresponding to the above predetermined positions in the amino acid sequences corresponding to SEQ ID NOs: 1 to 6 in any other SARS-CoV-2 are mutated) and mutant strains of viruses other than SARS-CoV-2 included in the Betacoronavirus genus (i.e., mutant strains in which amino acid residues corresponding to the above predetermined positions in the amino acid sequences corresponding to SEQ ID NOs: 1 to 6 in viruses other than SARS-CoV-2 included in the Betacoronavirus genus are mutated).

Each amino acid sequence corresponding to SEQ ID NOs: 1 to 6 in other betacoronavirus mutant strains is allowed to differ from the amino acid sequences set forth in SEQ ID NOs: 1 to 6, unless it significantly affects the properties of the polypeptides. The phrase "does not significantly affect the properties of the polypeptide" refers to a state in which a function as structural protein(s) and/or non-structural protein(s) of each polypeptide is maintained. Specifically, at a site other than amino acid residues corresponding to the mutations in SEQ ID NOs: 1 to 6 described above (hereinafter also referred to as an "any difference site"), a difference from SEQ ID NOs: 1 to 6 is acceptable. The acceptable difference may be one type of difference selected from substitution, addition, insertion, and deletion (e.g., substitution), or may include two or more types of differences (e.g., substitution and insertion). A sequence identity calculated by comparing only any difference sites of the amino acid sequences corresponding to SEQ ID NOs: 1 to 6 described above in any other SARS-CoV-2 and the amino acid sequences set forth in SEQ ID NOs: 1 to 6 may be 50% or more. In the any other SARS-CoV-2, the sequence identity is preferably 60% or more or 70% or more, more preferably 80% or more, further preferably 85% or more or 90% or more, still more preferably 95% or more, 96% or more, 97% or more, or 98% or more, still more preferably 99% or more, and particularly preferably 99.3% or more, 99.5% or more, 99.7% or more, or 99.9% or more. In any other betacoronaviruses, the sequence identity preferably includes 60% or more. Here, the "sequence identity" refers to a value of identity of an amino acid sequence obtained by bl2seq program of BLASTPACKAGE [sgi32 bit edition, Version 2.0.12; available from National Center for Biotechnology Information (NCBI)] (Tatiana A. Tatsusova, Thomas L. Madden, FEMS Microbiol. Lett., Vol. 174, p247?250, 1999). Parameters may be set to Gap insertion Cost value: 11 and Gap extension Cost value: 1.

In other words, the betacoronavirus temperature-sensitive strain (cold-adapted strain) of the present invention is more specifically as follows:
A betacoronavirus temperature-sensitive (cold-adapted) strain containing structural protein(s) and/or non-structural protein(s) consisting of at least any one of the following polypeptides (I-1) to (I-6), (II), and (III):
(I-1) a polypeptide (NSP3) consisting of an amino acid sequence having mutation(s) of at least any one of a mutation of valine at position 404 (a'), a mutation of leucine at position 445 (b'), a mutation of lysine at position 1792 (c'), and a mutation of aspartic acid at position 1832 (d') in an amino acid sequence set forth in SEQ ID NO: 1,
(I-2) a polypeptide (NSP14) consisting of an amino acid sequence having mutation(s) of at least any one of a mutation of glycine at position 248 (e'), a mutation of glycine at position 416 (f), and a mutation of alanine at position 504 (g') in an amino acid sequence set forth in SEQ ID NO: 2,
(I-3) a polypeptide (NSP16) consisting of an amino acid sequence having a mutation of valine at position 67 (h') in an amino acid sequence set forth in SEQ ID NO: 3,
(I-4) a polypeptide (spike) consisting of an amino acid sequence having mutation(s) of at least any one of a mutation of leucine at position 54 (i'), a mutation of threonine at position 739 (j'), and a mutation of alanine at position 879 (k') in an amino acid sequence set forth in SEQ ID NO: 4,
(I-5) a polypeptide (envelope) consisting of an amino acid sequence having a mutation of leucine at position 28 (l') in an amino acid sequence set forth in SEQ ID NO: 5, and
(I-6) a polypeptide (nucleocapsid) consisting of an amino acid sequence having a mutation of serine at position 2 (m') in an amino acid sequence set forth in SEQ ID NO: 6;
(II) a polypeptide wherein in the amino acid sequence of the polypeptides (1-1) to (I-6), one or more amino acid residues other than the amino acid residues related to the mutations (a') to (m') are substituted, added, inserted, or deleted, and the polypeptide constituting a betacoronavirus that has acquired a temperature-sensitive (cold adaptation) capability; and
(III) a polypeptide having a sequence identity of 50% or more in the amino acid sequences of the polypeptides (1-1) to (I-6) excluding the amino acid residues related to the mutations (a') to (m'), and constituting a betacoronavirus that has acquired a temperature-sensitive (cold adaptation) capability.

The above mutations of (a') to (m') refer to mutations when the mutations of (a) to (m) are specifically present in the amino acid sequence of SEQ ID NOs: 1 to 6, respectively. In other words, the above polypeptides (I-1) to (I-6) are polypeptides obtained by introducing mutation(s) of at least any one of the mutations (a') to (m') into polypeptides consisting of the amino acid sequences of SEQ ID NOs: 1 to 6 possessed by SARS-CoV-2 of NC_045512 (NCBI). In addition, the above polypeptides (II) and (III) are obtained by introducing mutation(s) of at least any one of the above mutations (a) to (m) into polypeptides consisting of amino acid sequences corresponding to the amino acid sequence of SEQ ID NOs: 1 to 6 possessed by another betacoronavirus. Preferred ranges of the sequence identity of the above polypeptides (II) and (III) are as described above.

Having such mutations, the betacoronavirus can acquire a temperature-sensitive (cold-adapted) property. In the virus temperature-sensitive strain (cold-adapted strain) of the present invention, a growth capability at a human lower respiratory tract temperature is at least decreased as compared with a growth capability at a temperature lower than a human lower respiratory tract temperature, and preferably, the virus temperature-sensitive strain (cold-adapted strain) of the present invention does not have a growth capability at a human lower respiratory tract temperature. In the present invention, the temperature-sensitive (cold adaptation) capability can be confirmed by the fact that a virus titer (TCID50/mL) in culture supernatants after Vero cells are infected with the virus temperature-sensitive strain at MOI = 0.01 at a human lower respiratory tract temperature and then the virus temperature-sensitive strain is cultured for 1 day at a human lower respiratory tract temperature is decreased, for example, by 10² or more, preferably by 10³ or more, as compared with a virus titer in culture supernatants after Vero cells are infected with the virus temperature-sensitive strain at MOI = 0.01 at a human upper respiratory tract temperature and then the virus temperature-sensitive strain is cultured for 1 day at a human upper respiratory tract temperature.

Typically, in the virus temperature-sensitive strain (cold-adapted strain) of the present invention, a growth capability at a human lower respiratory tract temperature is decreased as compared with a growth capability at a human lower respiratory tract temperature in the case of not having any of the above mutations of (a) to (m). This can be confirmed by the fact that a virus titer (TCID50/mL) in culture supernatants after Vero cells are infected with the virus temperature-sensitive strain at MOI = 0.01 at a human lower respiratory tract temperature and then the virus temperature-sensitive strain is cultured for 1 day at a human lower respiratory tract temperature is decreased, for example, by 10² or more, preferably by 10³ or more, as compared with a virus titer in culture supernatants after Vero cells are infected with a strain not having any of the above mutations of (a) to (m) at MOI = 0.01 at a human lower respiratory tract temperature and then the strain is cultured for 1 day at a human lower respiratory tract temperature.

Representative examples of the human lower respiratory tract temperature include about 37°C, and specifically include a temperature higher than the upper respiratory tract temperature described below, preferably 36 to 38°C, and more preferably 36.5 to 37.5°C or 37 to 38°C. In addition, the virus temperature-sensitive strain (cold-adapted strain) of the present invention may have a growth capability at a temperature lower than a human lower respiratory tract temperature. For example, the temperature lower than the human lower respiratory tract temperature may include, for example, a human upper respiratory tract temperature (as a specific example, about 32°C to 35.5°C).

The above mutations of (a) to (m) are not present on a receptor-binding domain of a spike protein present on a surface of a virus, which is important when the virus infects cells. Therefore, it is reasonably expected that not only the SARS-CoV-2 listed in NC_045512 (NCBI) but also other betacoronaviruses can be made temperature-sensitive by introducing at least any one of the above mutations of (a) to (m). In other words, even if a mutation occurs that alters the immunogenicity of the virus due to worldwide infection, it is reasonably expected that temperature sensitivity can be imparted to the mutant virus by further introducing at least any one of the above mutations of (a) to (m) into the mutant virus.

The above mutation of (a) may be a substitution with an amino acid residue other than valine, the above mutation of (b) may be a substitution with an amino acid residue other than leucine, the above mutation of (c) may be a substitution with an amino acid residue other than lysine, the above mutation of (d) may be a substitution with an amino acid residue other than aspartic acid, the above mutation of (e) may be a substitution with an amino acid residue other than glycine, the above mutation of (f) may be a substitution with an amino acid residue other than glycine, the above mutation of (g) may be a substitution with an amino acid residue other than alanine, the above mutation of (h) may be a substitution with an amino acid residue other than valine, the above mutation of (i) may be a substitution with an amino acid residue other than leucine, the above mutation of (j) may be a substitution with an amino acid residue other than threonine, the above mutation of (k) may be a substitution with an amino acid residue other than alanine, the above mutation of (1) may be a substitution with an amino acid residue other than leucine, and the above mutation of (m) may be a substitution with an amino acid residue other than serine.

In a preferred example of the virus temperature-sensitive strain (cold-adapted strain) of the present invention, the mutation of (a) is a substitution with alanine, the mutation of (b) is a substitution with phenylalanine, the mutation of (c) is a substitution with arginine, the mutation of (d) is a substitution with asparagine, the mutation of (e) is a substitution with valine, the mutation of (f) is a substitution with serine, the mutation of (g) is a substitution with valine, the mutation of (h) is a substitution with isoleucine, the mutation of (i) is a substitution with tryptophan, the mutation of (j) is a substitution with lysine, the mutation of (k) is a substitution with valine, the mutation of (1) is a substitution with proline, and/or the mutation of (m) is a substitution with phenylalanine.

In another example of the virus temperature-sensitive strain (cold-adapted strain) of the present invention, the substitution may be a so-called conservative replacement. The conservative replacement refers to a substitution with an amino acid having a similar structure and/or property, and examples of the conservative replacement include a substitution with another non-polar amino acid if the amino acid before substitution is a non-polar amino acid, a substitution with another non-charged amino acid if the amino acid before substitution is a non-charged amino acid, a substitution with another acidic amino acid if the amino acid before substitution is an acidic amino acid, and a substitution with another basic amino acid if the amino acid before substitution is a basic amino acid. In general, the "non-polar amino acid" includes alanine, valine, leucine, isoleucine, proline, methionine, phenylalanine, and tryptophan, the "non-charged amino acid" includes glycine, serine, threonine, cysteine, tyrosine, asparagine, and glutamine, the "acidic amino acid" includes aspartic acid and glutamic acid, and the "basic amino acid" includes lysine, arginine, and histidine.

A more preferred example of the virus temperature-sensitive strain (cold-adapted strain) of the present invention is a mutant strain of the SARS-CoV-2 listed in NC_045512 (NCBI), wherein the mutation of (a) (i.e., the mutation of (a')) is a substitution of valine at position 404 of an amino acid sequence set forth in SEQ ID NO: 1 with alanine in NSP3 (V404A); the mutation of (b) (i.e., the mutation of (b')) is a substitution of leucine at position 445 of an amino acid sequence set forth in SEQ ID NO: 1 with phenylalanine in NSP3 (L445F); the mutation of (c) (i.e., the mutation of (c')) is a substitution of lysine at position 1792 in an amino acid sequence set forth in SEQ ID NO: 1 with arginine in NSP3 (K1792R); the mutation of (d) (i.e., the mutation of (d')) is a substitution of aspartic acid at position 1832 of an amino acid sequence set forth in SEQ ID NO: 1 with asparagine in NSP3 (D1832N); the mutation of (e) (i.e., the mutation of (e')) is a substitution of glycine at position 248 of an amino acid sequence set forth in SEQ ID NO: 2 with valine in NSP14 (G248V); the mutation of (f) (i.e., the mutation of (f)) is a substitution of glycine at position 416 of an amino acid sequence set forth in SEQ ID NO: 2 with serine in NSP14 (G416S); the mutation of (g) (i.e., the mutation of (g')) is a substitution of alanine at position 504 of an amino acid sequence set forth in SEQ ID NO: 2 with valine in NSP14 (A504V); the mutation of (h) (i.e., the mutation of (h')) is a substitution of valine at position 67 of an amino acid sequence set forth in SEQ ID NO: 3 with isoleucine in NSP16 (V67I); the mutation of (i) (i.e., the mutation of (i')) is a substitution of leucine at position 54 of an amino acid sequence set forth in SEQ ID NO: 4 with tryptophan in a spike (L54W); the mutation of (j) (i.e., the mutation of (j')) is a substitution of threonine at position 739 of an amino acid sequence set forth in SEQ ID NO: 4 with lysine in a spike (T739K); the mutation of (k) (i.e., the mutation of (k')) is a substitution of alanine at position 879 of an amino acid sequence set forth in SEQ ID NO: 4 with valine in a spike (A879V); the mutation of (1) (i.e., the mutation of (l')) is a substitution of leucine at position 28 of an amino acid sequence set forth in SEQ ID NO: 5 with proline in an envelope (L28P); and/or the mutation of (m) (i.e., the mutation of (m')) is a substitution of serine at position 2 of an amino acid sequence set forth in SEQ ID NO: 6 with phenylalanine in a nucleocapsid (S2F).

The betacoronavirus temperature-sensitive (cold-adapted) strain of the present invention may further have a deletion of an amino acid sequence encoded by a base sequence set forth in SEQ ID NO: 7. The base sequence set forth in SEQ ID NO: 7 is a part of an open reading frame of theSARS-CoV-2, NC_045512 (NCBI).

Particularly preferred examples of the virus temperature-sensitive strain (cold-adapted strain) of the present invention include the following strains.
· A strain having the mutation of (e) (preferably the mutation of (e') and/or G248V), the mutation of (f) (preferably the mutation of (f) and/or G416S), the mutation of (g) (preferably the mutation of (g') and/or A504V), the mutation of (k) (preferably the mutation of (k') and/or A879V), the mutation of (1) (preferably the mutation of (l') and/or L28P), and the mutation of (m) (preferably the mutation of (m') and/or S2F)
· A strain having the mutation of (a) (preferably the mutation of (a') and/or V404A), the mutation of (d) (preferably the mutation of (d') and/or D1832N), the mutation of (h) (preferably the mutation of (h') and/or V67I), and the mutation of (j) (preferably the mutation of (j') and/or T739K); or a strain further having a deletion of an amino acid sequence encoded by a base sequence set forth in SEQ ID NO: 7.
· A strain having the mutation of (b) (preferably the mutation of (b') and/or L445F) and the mutation of (c) (preferably the mutation of (c') and/or K1792R): or a strain further having a deletion of an amino acid sequence encoded by a base sequence set forth in SEQ ID NO: 7.
· A strain having the mutation of (b) (preferably the mutation of (b') and/or L445F), the mutation of (c) (preferably the mutation of (c') and/or K1792R), and the mutation of (i) (preferably the mutation of (i') and/or L54W); or a strain further having a deletion of an amino acid sequence encoded by a base sequence set forth in SEQ ID NO: 7.

### 2. Vaccine

### 2-1. Active ingredient of attenuated vaccine

As described above, since the betacoronavirus temperature-sensitive strain (cold-adapted strain) described in "1. Betacoronavirus temperature-sensitive strain (cold-adapted strain)" can efficiently proliferate only at a temperature lower than a human lower respiratory tract temperature, it can be expected that it cannot efficiently proliferate at least in a deep part of a living body, especially in the lower respiratory tract including the lung that causes serious disorders, and pathogenicity is significantly decreased. Therefore, the virus temperature-sensitive strain (cold-adapted strain) can be used as a live attenuated vaccine by infecting a living body as an attenuated virus itself. Therefore, the present invention also provides a vaccine containing the above betacoronavirus temperature-sensitive strain (cold-adapted strain) as an active ingredient. Details of the active ingredient are as described in "1. Betacoronavirus temperature-sensitive strain (cold-adapted strain)".

### 2-2. Active ingredient of gene-based vaccine

As described in the above "1. Betacoronavirus temperature-sensitive strain (cold-adapted strain)", the predetermined mutation contributes to imparting a temperature-sensitive (cold adaptation) capability. Therefore, the present invention also provides a betacoronavirus gene-based vaccine containing, as an active ingredient, a gene encoding structural protein(s) and/or non-structural protein(s) having at least any one of the above mutations of (a) to (m). Details of the mutation contained in the active ingredient are as described in "1. Betacoronavirus temperature-sensitive strain (cold-adapted strain)".

### 2-3. Targeted viruses

The vaccine of the present invention can be reasonably expected to be effective against not only the early Wuhan strain of SARS-CoV-2 but also a wide range of SARS-CoV-2 virus-associated strains including the variants detected in the United Kingdom in September 2020 and detected in South Africa in October 2020, and other known variants, as well as unknown variants yet to be detected, and viruses other than SARS-CoV-2 included in the Betacoronavirus genus. Therefore, the vaccine of the present invention targets betacoronaviruses.

### 2-4. Other components

The vaccine of the present invention can contain other components such as adjuvants, buffer, tonicity agents, soothing agents, preservatives, antioxidants, flavoring agents, light-absorbing dye, stabilizers, carbohydrate, casein digest, and various vitamins, in addition to the above active ingredients, according to the purpose, use, and the like.

Examples of the adjuvants include animal oils (squalene and the like) or hardened oils thereof; vegetable oils (palm oil, castor oil, and the like) or hardened oils thereof; oily adjuvants including anhydrous mannitol/oleic acid ester, liquid paraffin, polybutene, caprylic acid, oleic acid, higher fatty acid ester, and the like; water-soluble adjuvants such as PCPP, saponin, manganese gluconate, calcium gluconate, manganese glycerophosphate, soluble aluminum acetate, aluminum salicylate, acrylic acid copolymer, methacrylic acid copolymer, maleic anhydride copolymer, alkenyl derivative polymer, oil-in-water emulsion, and cationic lipid containing quaternary ammonium salt; precipitating adjuvants including aluminum salts such as aluminum hydroxide (alum), aluminum phosphate, and aluminum sulfate or combinations thereof, sodium hydroxide, and the like; microorganism-derived toxin components such as cholera toxin and *E. coli* heat-labile toxin; and other components (bentonite, muramyl dipeptide derivative, interleukin, and the like).

Examples of the buffers include buffers such as phosphate, acetate, carbonate, and citrate. Examples of the isotonizing agents include sodium chloride, glycerin, and D-mannitol. Examples of the soothing agents include benzyl alcohol. Examples of the preservatives include thimerosal, para-hydroxybenzoates, phenoxyethanol, chlorobutanol, benzyl alcohol, phenethyl alcohol, dehydroacetic acid, sorbic acid, antibiotics, and synthetic antibacterial agents. Examples of the antioxidants include sulfite and ascorbic acid.

Examples of the light-absorbing dye include riboflavin, adenine, and adenosine. Examples of the stabilizers include chelating agents and reducing agents. Examples of the carbohydrate include sorbitol, lactose, mannitol, starch, sucrose, glucose, and dextran.

Furthermore, the vaccine of the present invention may contain one or more other vaccines against viruses or bacteria that cause other diseases other than betacoronavirus infection, such as COVID-19. In other words, the vaccine of the present invention may be prepared as a combination vaccine containing other vaccines.

### 2-5. Dosage forms

A dosage form of the vaccine of the present invention is not particularly limited, and can be appropriately determined based on an administration method, storage conditions, and the like. Specific examples of the dosage form include liquid preparations and solid preparations, and more specifically, oral administration agents such as tablets, capsules, powders, granules, pills, solutions, and syrups; and parenteral administration agents such as injections and sprays.

### 2-6. Administration methods

A method for administering the vaccine of the present invention is not particularly limited, and may be any of injection administration such as intramuscular, intraperitoneal, intradermal, and subcutaneous administration; inhalation administration from the nasal cavity and the oral cavity; oral administration, and the like, but injection administration such as intramuscular, intradermal, and subcutaneous administration (intramuscular administration, intradermal administration, and subcutaneous administration) and inhalation administration from the nasal cavity (nasal administration) are preferable, and nasal administration is more preferable.

### 2-7. Applicable subjects

A subject to which the vaccine of the present invention is applied is not particularly limited as long as the subject that can develop various symptoms by betacoronavirus infection (preferably a subject that can develop COVID-19 symptoms by SARS-CoV-2 infection), and examples thereof include mammals, and more specifically, humans; pet animals such as dogs and cats; and experimental animals such as rats, mice, and hamsters.

### 2-8. Dose

A dose of the vaccine of the present invention is not particularly limited, and can be appropriately determined according to the type of an active ingredient, an administration method, and a subject receiving administration (conditions such as age, weight, sex, and presence or absence of underlying disease). For example, a dose for a human includes 1 × 10¹⁰ TCID50/kg or less, preferably 1 × 10⁸ TCID50/kg or less.

### 3. Method for producing betacoronavirus temperature-sensitive strain (cold-adapted strain)

A method for producing the betacoronavirus temperature-sensitive strain (cold-adapted strains) of the present invention is not particularly limited, and can be appropriately determined by those skilled in the art based on the above amino acid sequence information. For example, from the viewpoint of producing a vaccine that is relatively inexpensive and has little lot difference, a reverse genetics method using an artificial chromosome such as bacterial artificial chromosome (BAC) or yeast artificial chromosome (YAC), or CPER method or the like using a genomic fragment of a betacoronavirus is preferable.

In a method for reconstituting a virus by the reverse genetics method, first, a genome of a strain (parent strain) not having any of the mutations of (a) to (m) of the betacoronavirus temperature-sensitive strain (cold-adapted strain) is cloned. The parent strain used at this time may be a betacoronavirus, and specifically, it can be selected from the group consisting of the above SARS-CoV-2 listed in NC_045512 (NCBI), the above any other SARS-CoV-2, and viruses other than SARS-CoV-2 included in the Betacoronavirus genus.

Furthermore, when an artificial chromosome is used in the reverse genetics method, full-length DNA of a viral genome is cloned into BAC DNA, YAC DNA, or the like, and a transcription promoter sequence for eukaryotic cells is inserted upstream of a sequence of the virus. Examples of the promoter sequence include CMV promoter and CAG promoter. A ribozyme sequence and a polyA sequence are inserted downstream of a sequence of the virus. Examples of the ribozyme sequence include a hepatitis D virus ribozyme and a hammer head ribozyme. Examples of the polyA sequence include polyA of Simian 40 virus.

On the other hand, when the CPER method is used in the reverse genetics method, full-length DNA of a viral genome is divided into several fragments and cloned. Examples of a method for acquiring fragments include a method for artificially synthesizing nucleic acid and a PCR method using a plasmid obtained by cloning the above artificial chromosome or fragments as a template.

In order to introduce at least any one of the above mutations of (a) to (m) into the viral genome cloned by the methods described above, a known point mutation introduction method such as a homologous recombination method such as double crossover or λ/RED recombination, an overlap PCR method, or a CRISPR/Cas9 method can be used.

Subsequently, the artificial chromosome into which mutations have been introduce is transfected into a host cell to reconstitute a recombinant virus. In the case of the reverse genetics method by the CPER method, fragments into which mutations have been introduced are assembled by a reaction using a DNA polymerase, and then transfected into host cells to reconstitute a recombinant virus. The method for transfection is not particularly limited, and a known method can be used. The host is also not particularly limited, and known cells can be used.

Subsequently, the reconstituted recombinant virus is infected to cultured cells to passage the recombinant virus. The cultured cells used at that time are not particularly limited, and examples thereof include Vero cells, VeroE6 cells, Vero cells supplementing expression of TMPRESS2, VeroE6 cells supplementing expression of TMPRESS2, Calu-3 cells, 293T cells supplementing expression of ACE2, BHK cells, 104C1 cells, and mouse neuroblastoma-derived NA cells. The virus can be recovered by a known method such as centrifugation or membrane filtration. In addition, mass production of a recombinant virus becomes possible by further adding the recovered virus to cultured cells.

### EXAMPLES

Hereinafter, the present invention will be described in detail with reference to Examples, but the present invention is not limited thereto.

### [Test Example 1]

### [Test Example 1-1] Isolation of a temperature sensitive strain (cold-adapted strain) of SARS-CoV-2, A50-18 strain

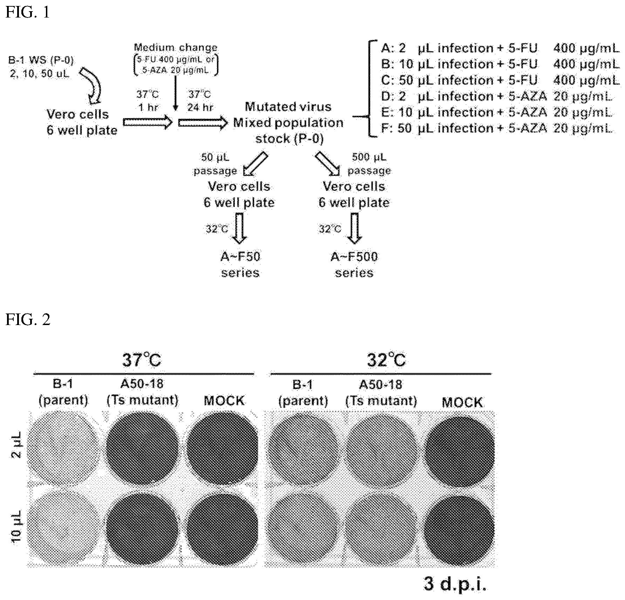

Based on the schematic diagram in Fig. 1, two types of mutation inducers 5-fluorouracil (hereinafter, 5-FU) and 5-azacytidine (hereinafter, 5-AZA) were exposed to a clinical isolate of SARS-CoV-2 (hereinafter, B-1 strain) to obtain virus populations of A to F50 series and A to F500 series that were adapted at 32°C. Furthermore, the mutated virus populations passaged at several times, and 406 candidate strains were isolated from these series, and temperature-sensitive strain (A50-18 strain. Hereinafter, the strain may be referred to as a Ts srain) which can growth at 32°C but has significantly decreased growth at 37°C, was isolated and selected from among the candidates (Fig. 2).

### [Test Example 1-2] Mutation analysis of temperature-sensitive strain (cold-adapted strain), A50-18 strain, by next-generation sequencing

### (1-2-1) Mutation analysis of each virus strain

Mutation analysis of the following virus strains was performed using a next-generation sequencer. The analysis was performed by extracting RNA from culture supernatants of Vero cells infected with SARS-CoV-2. As a reference, Wuhan-Hu-1 (NC045512), a Wuhan clinical isolate, was used.
B-1: Wild strain (clinical isolate)
A50-18: Temperature-sensitive strain (cold-adapted strain)
F50-37: Non-temperature-sensitive strain
C500-1: Non-temperature-sensitive strain
F500-53: Non-temperature-sensitive strain
F500-40: Non-temperature-sensitive strain
F500-2: Non-temperature-sensitive strain
(Viruses other than B-1 were mutation induced strain.)

### (1-2-2) Mutations of a temperature-sensitive strain (cold-adapted strain)

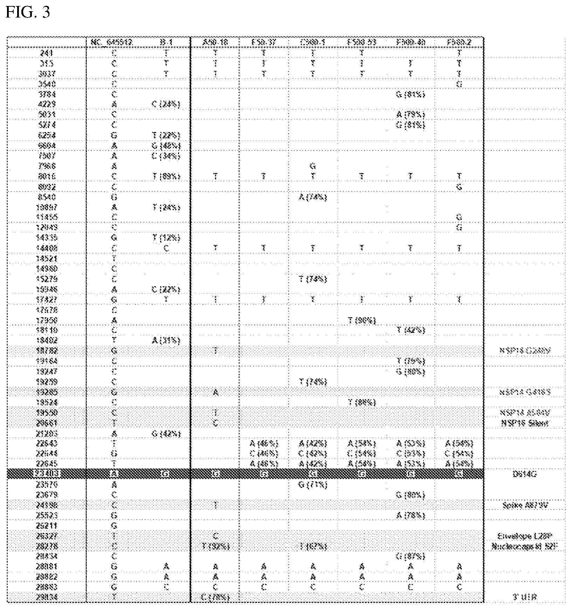

Fig.3 shows the analysis result from (1-2-1). Since a point mutation of D614G was also observed in the B-1 strain, the point mutation was not specific for the temperature-sensitive strain (cold-adapted strain). On the other hand, as characteristic point mutations of the temperature-sensitive strain (cold-adapted strain) (A50-18), G248V, G416S, and A504V in NSP14, A879V in spike, L28P in envelope, and S2F in nucleocapsid were identified.

### (1-2-3) Analysis of temperature-sensitive strains (cold-adapted strains) by Sanger sequencing

Mutation analysis of the A50-18 strain was performed using Sanger sequencing. The analysis was performed by extracting RNA from culture supernatants of Vero cells infected with the SARS-CoV-2. As a result, no deletion as observed in (2-2-3) of Test Example 2-2 described later was found.

### (1-2-4) Summary of mutations of temperature-sensitive strains (cold-adapted strains)

In the temperature-sensitive strain (cold-adapted strain), the A50-18 strain, as shown in Table 2 below, mutations with check marks in the amino acid sequences of the SEQ ID NO indicated were found.

**[Table 2]**

| Mutation sign | Polypeptide | SEQ ID NO | Amino acid before mutation | Mutation position | Amino acid after mutation | A50-18 (Ts) |
|---|---|---|---|---|---|---|
| (a') | NSP3 | 1 | V | 404 | A | |
| (b') | NSP3 | 1 | L | 445 | F | |
| (c') | NSP3 | 1 | K | 1792 | R | |
| (d') | NSP3 | 1 | D | 1832 | N | |
| (e') | NSP14 | 2 | G | 248 | V | ✔ |
| (f') | NSP14 | 2 | G | 416 | S | ✔ |
| (g') | NSP14 | 2 | A | 504 | V | ✔ |
| (h') | NSP16 | 3 | V | 67 | I | |
| (i') | Spike | 4 | L | 54 | w | |
| (j') | Spike | 4 | T | 739 | K | |
| (k') | Spike | 4 | A | 879 | V | ✔ |
| (l') | Envelope | 5 | L | 28 | P | ✔ |
| (m') | Nucleocapsid | 6 | S | 2 | F | ✔ |
| | ORF | Deletion of amino acid sequence encoded by base sequence of SEQ ID NO: 7 | | | | |

### [Test Example 1-3] Growth dynamic of temperature-sensitive strain (cold-adapted strain), A50-18 strain

### (1-3-1) Analysis at 32°C and 37°C

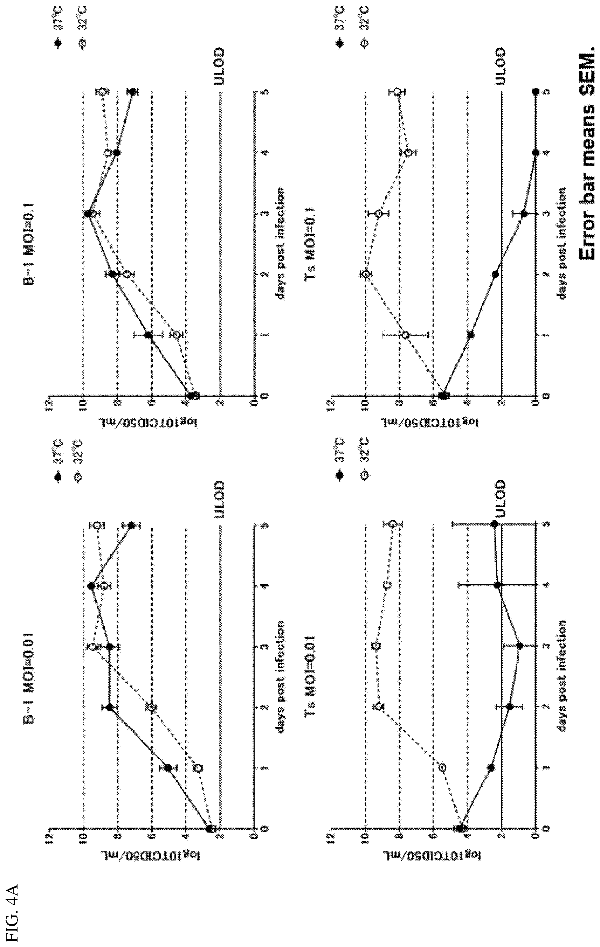

Vero cells were infected with a clinical isolate (B-1 strain) and a temperature-sensitive strain (cold-adapted strain) (A50-18 strain) using 6-well plates under conditions of MOI = 0.01 or 0.1 (N = 3). The viruses cultured at 37°C or 32°C, and each culture supernatant was collected at 0 to 5 dpi. Viral titers of culture supernatants at 0 to 5 dpi were evaluated by at TCID50/mL using the Vero cells. The results were shown in Fig. 4A.

From Fig. 4 A, it was found that the virus titer at 3 days after infection at 37°C was under the limit of detection in the A50-18 strain, and growth at 37°C was significantly decreased.

### (1-3-2) Growth dynamics at 32°C, 34°C, and 37°C

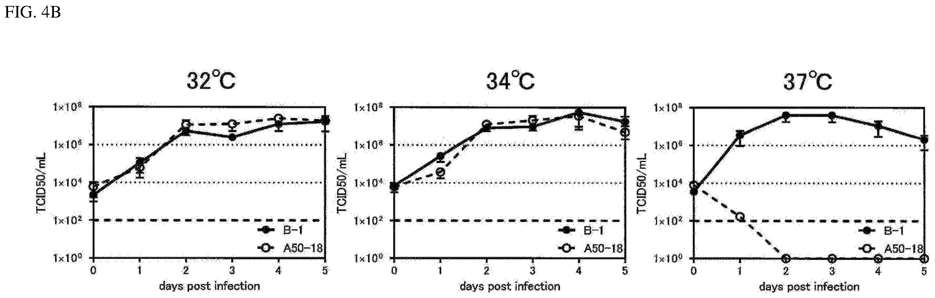

Vero cells were infected with a clinical isolate (B-1 strain) and a temperature-sensitive strain (cold-adapted strain) (A50-18 strain) using 6-well plates under conditions of MOI = 0.01 (N = 3). The viruses cultured at 37°C, 34°C, or 32°C, and each culture supernatant was collected at 0 to 5 dpi. Viral titers of culture supernatants at 0 to 5 dpi were evaluated by TCID50/mL using the Vero cells. The results were shown in Fig. 4B.

From Fig. 4B, it was found that the A50-18 strain proliferated comparably to the clinical isolate at 32°C and 34°C, while growth at 37°C was significantly decreased.

### [Test Example 1-4] Evaluation of pathogenicity of temperature-sensitive strain (cold-adapted strain), A50-18 strain

### (1-4-1) Weight changes of SARS-CoV-2-infected hamsters

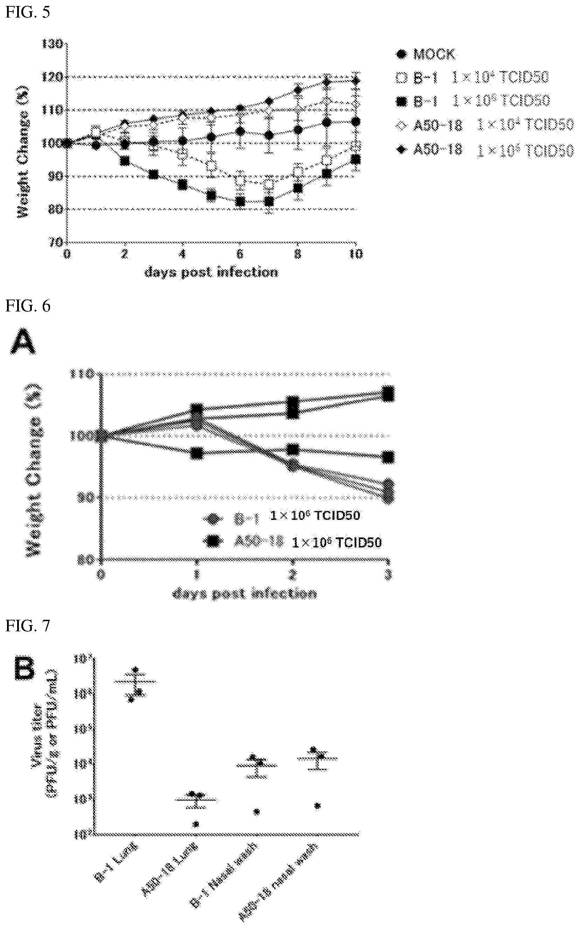

4-week-old male Syrian hamsters (n = 4) were bred for 1 week, and then the clinical isolate (B-1 strain) and the temperature-sensitive strain (cold-adapted strain) (A50-18 strain) (1 × 10⁴ or 1 × 10⁶ TCID50) were nasally administered in a volume of 100 µL, and weight changes for 10 days were monitored. The group to which the same volume of D-MEM medium was nasally administered was defined as a non-infected control (MOCK). The results were shown in Fig. 5. A50-18 strain-infected hamsters did not lose weight, suggesting that this strain had significant lower pathogenicity.

### (1-4-2) Evaluation of viral loads in the lungs and nasal wash specimens of SARS-CoV-2-infected hamsters

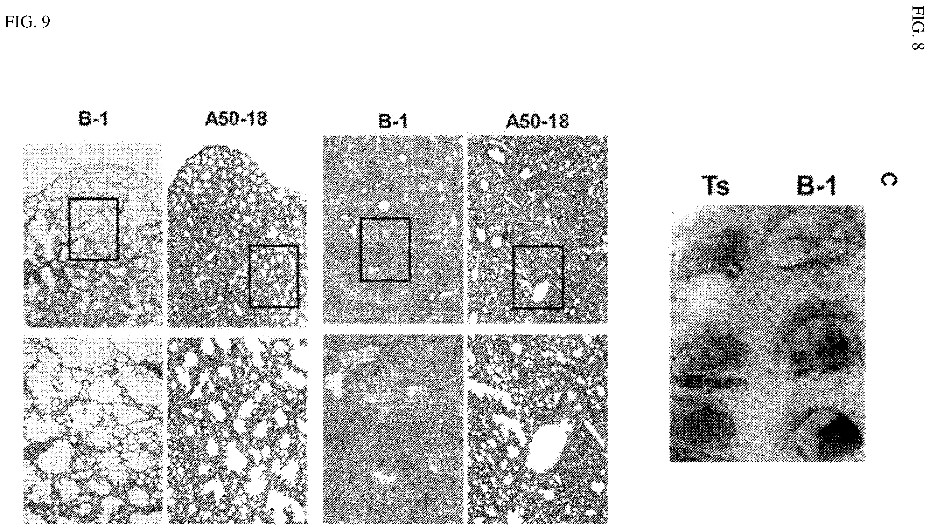

4-week-old male Syrian hamsters (n = 3) were bred for 1 week, and then the clinical isolate (B-1 strain) and the temperature-sensitive strain (cold-adapted strain) (A50-18 strain) (1 × 10⁶ TCID50) were nasally administered in a volume of 100 µL. The results of observing the weight changes for 3 days were shown in Fig. 6. The hamsters were euthanized at 3 dpi, and then nasal wash specimens were collected with 1 mL of D-PBS. In addition, lungs of the hamsters were extracted, the right lungs were homogenized, and suspended with 1 mL of D-MEM, and then the supernatants were recovered as lung homogenates by centrifugation. The results of evaluating the viral loads in these nasal wash specimens and lung homogenates by plaque formation assay using Vero cells were shown in Fig. 7. Furthermore, the extracted left lungs were fixed with 10% formalin and photographed, and the photographs were shown in Fig. 8.

From Fig. 7, it was found that there was no difference in the viral loads between the B-1 strain and the A50-18 strain in the nasal wash specimens, while the virus titer of the A50-18 strain was remarkably low in the lungs. In addition, from Fig. 8, it was found that hamsters infected with the B-1 strain, the weight of which were decreased, had swelling or blackening, whereas the lungs of hamsters infected with the A50-18 strain, that showed no weight change, and the lungs of the hamsters had no remarkable changes. From these results, it is estimated that the temperature-sensitive strain (cold-adapted strain) is an attenuated strain that proliferates in the upper respiratory tracts but cannot proliferate in the lower respiratory tracts.

### (1-4-3) Histological analysis of SARS-CoV-2-infected hamsters

Lung Sections were prepared from the formalin-fixed lungs obtained by the infection experiment to hamsters performed in (1-4-2), and HE staining was performed to analyze histological pathogenicity of the lungs by SARS-CoV-2 infection. The results were shown in Fig. 9.

As shown in Fig. 9, infiltration of erythrocytes and destruction of alveoli were observed in the lungs of the hamsters infected with the clinical isolate (B-1 strain). On the other hand, in the lungs of the hamsters infected with the temperature-sensitive strain (cold-adapted strain) (A50-18 strain), such severe pathologies were not observed. This also strongly suggested that the A50-18 strain did not induce severe inflammation in the lungs by infection and had low pathogenicity.

### (1-4-4) Histological analysis of SARS-CoV-2-infected hamsters by immunochemical staining

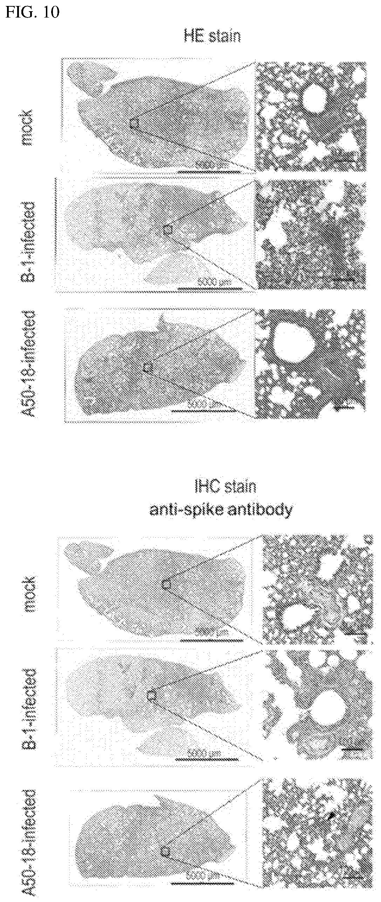

In order to evaluate relationships between virus growth and pathogenicity for the histological pathogenicity observed in (1-4-3), viral proteins were detected by immunochemical staining. 4-week-old male Syrian hamsters (B-1, A50-18: n = 5, MOCK: n = 3) were bred for 1 week, and then the clinical isolate (B-1 strain) and the temperature-sensitive strain (cold-adapted strain) (A50-18 strain) (1 × 10⁶ TCID50) were nasally administered in a volume of 100 µL. Hamsters were euthanized at 3 dpi, and then the extracted left lungs were fixed with 10% formalin to prepare serial sections. HE staining and immunochemical staining (also referred to as immunohistochemistry (IHC) staining) were performed on the obtained serial section. For immunochemical staining, rabbit anti-spike polyclonal antibody (Sino Biological, Inc.: 40589-T62) was used. HE staining images and immunochemical staining images are shown in Fig. 10. As in (1-4-3), in the hamsters infected with the B-1 strain, infiltration of erythrocytes and destruction of alveoli were observed, and spike proteins were widely detected by immunochemical staining. On the other hand, in the hamsters infected with the A50-18 strain, such tissue damage was not observed, and spike proteins were also locally detected only in limited areas. From these results, it was revealed that the B-1 strain exhibited tissue damage with remarkable virus growth in the lung tissue, whereas in the A50-18 strain, the virus could not efficiently proliferate in the lung tissue and lung tissue damage was low.

### [Test Example 1-5] Immunogenicity analysis of temperature-sensitive strain (cold-adapted strain), A50-18 strain

### (1-5-1) Wild-type strain challenge on hamsters infected with a temperature-sensitive strain (cold-adapted strain)

According to the following procedure, hamsters which infected with a temperature-sensitive strain (cold-adapted strain) were challenged with a wild type strain (clinical isolate)

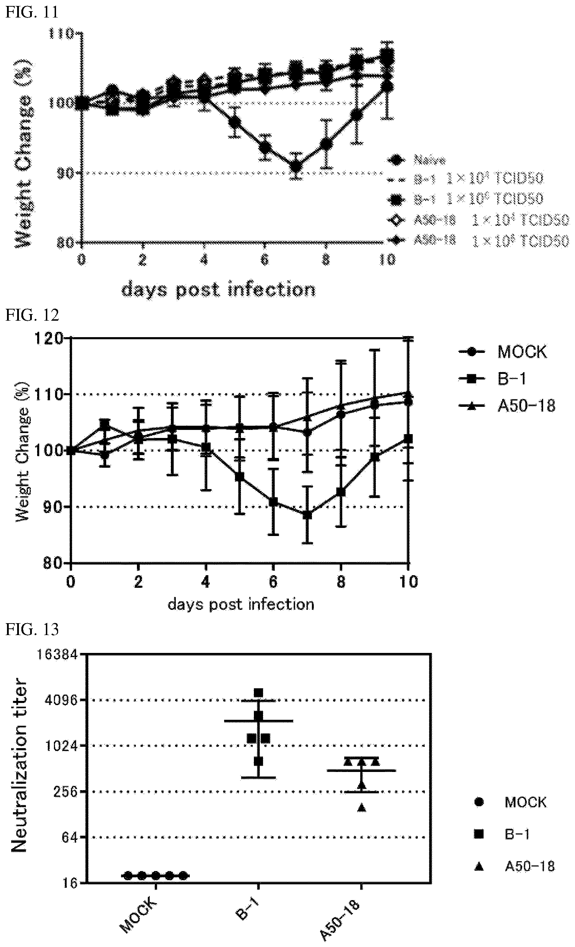

4-week-old male Syrian hamsters (n = 4) were bred for 1 week, and then the clinical isolate (B-1 strain) or the temperature-sensitive strain (cold-adapted strain) (A50-18 strain) (1 × 10⁴ or 1 × 10⁶ TCID50) was nasally administered in a volume of 100 µL. After 21 days, the clinical isolate (B-1 strain) (1 × 10⁶ TCID50) was nasally administered again in a volume of 100 µL, and weight changes for 10 days were monitored. At this time, naive hamsters (n = 3) of the same age were used as a naive control. The results were shown in Fig. 11.

As shown in Fig. 11, the naive hamsters lost weight by the infection with the B-1 strain, while the hamsters infected once with the B-1 strain or the A50-18 strain did not lose weight. From this, it was revealed that immunity contributing to infection protection can be induced not only by the infection of B-1 strain, which is a wild strain, but also by the infection of A50-18 strain, which shows low pathogenicity.

### (1-5-2) Evaluation of neutralizing antibody induced by hamsters infected with temperature-sensitive strain (cold-adapted strain)

4-week-old male Syrian hamsters (n = 5) were bred for 1 week, and then the clinical isolate (B-1 strain) or the temperature-sensitive strain (cold-adapted strain) (A50-18 strain) (1 × 10⁶ TCID50) was nasally administered in a volume of 100 µL. Weight changes after infection are shown in Fig. 12. Similar to the previous results, weight loss was observed by infection with the B-1 strain, while no weight loss was observed by infection with the A50-18 strain.

21 days post infection, whole blood was collected, serum was separated, and then serum was inactivated by heat at 56°C for 30 minutes. The B-1 strain of 100 TCID50 was mixed with serially diluted inactivated serum, and the mixture was incubated at 37°C for 1 hour. The culture solution after incubation added on Vero cells, and neutralizing activity of the virus was evaluated by observing CPE after culturing at 37°C. The highest dilution rate of serum which did not cause CPE, was defined as a neutralizing antibody titer. The results are shown in Fig. 13. It was revealed that serum of the non-infected hamsters did not show neutralizing activity against the B-1 strain, whereas serum of the hamsters infected with the B-1 strain or the A50-18 strain could show neutralizing activity.

### [Test Example 2]

### [Test Example 2-1] Additional isolation of SARS-CoV-2 temperature-sensitive strains (cold-adapted strains), H50-11 strain, L50-33 strain, and L50-40 strain

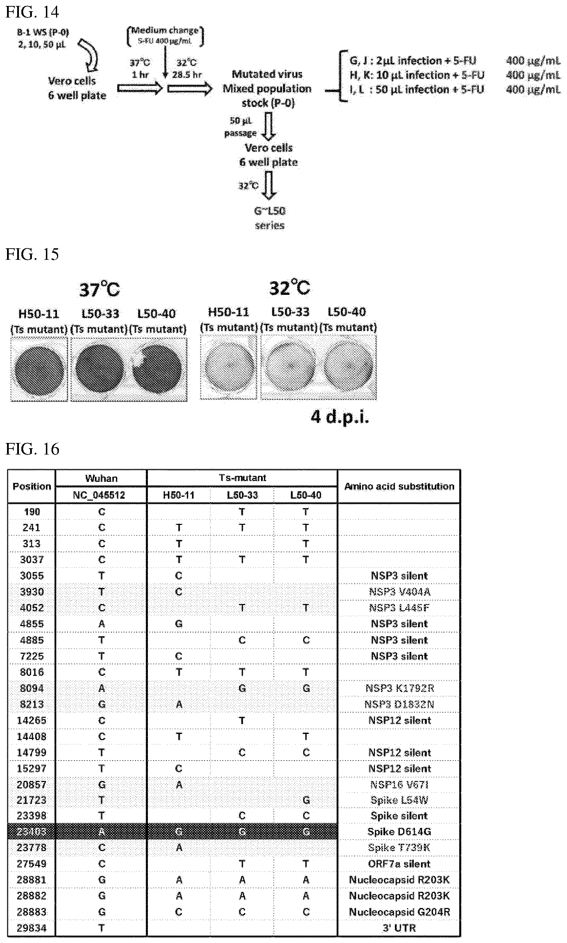

For the purpose of isolation of further candidate strains, temperature-sensitive strains (cold-adapted strains) were isolated by the method of Fig. 14. Vero cells were infected with a clinical isolate of SARS-CoV-2 (hereinafter, B-1 strain), and a mutation inducer 5-FU was added, a virus population of G to L50 series that were adapted at 32°C were obtained. Furthermore, passaging of each virus population was performed several times, and from among the obtained 253 strains, virus strains that can proliferate at 32°C but have significantly decreased growth at 37°C (H50-11 strain, L50-33 strain, and L50-40 strain) were found, isolated, and selected (Fig. 15).

### [Test Example 2-2] Analysis of additional temperature-sensitive (cold-adapted) isolates, H50-11 strain, L50-33 strain, and L50-40 strain, by next-generation sequencing (2-2-1) Mutation analysis method of additional isolates

Mutation analysis of the following virus strains was performed using a next-generation sequencer. The analysis was performed by extracting RNA from culture supernatants of Vero cells infected with SARS-CoV-2. As a reference, Wuhan-Hu-1 (NC045512), a Wuhan clinical isolate, was used.
H50-11 strain: Temperature-sensitive strain (cold-adapted strain)
L50-33 strain: Temperature-sensitive strain (cold-adapted strain)
L50-40 strain: Temperature-sensitive strain (cold-adapted strain)

### (2-2-2) Results of mutation analysis of temperature-sensitive strains (cold-adapted strains)

From (2-2-1), the analysis results of Fig. 16 were obtained. As characteristic point mutations of the H50-11 strain, V404A and D1832N in NSP3, V67I in NSP16, and T739K in spike were identified. In addition, as characteristic point mutations of the L50-33 strain, L445F and K1792R in NSP3 were found, and as characteristic point mutations of the L50-40 strain, L445F and K1792R in NSP3 and L54W in spike were found.

### (2-2-3) Evaluation of temperature-sensitive strains (cold-adapted strains) by Sanger sequencing

Mutation analysis of the H50-11 strain, the L50-33 strain, and the L50-40 strain was performed using Sanger sequencing. The analysis was performed by extracting RNA from culture supernatants of Vero cells infected with SARS-CoV-2.

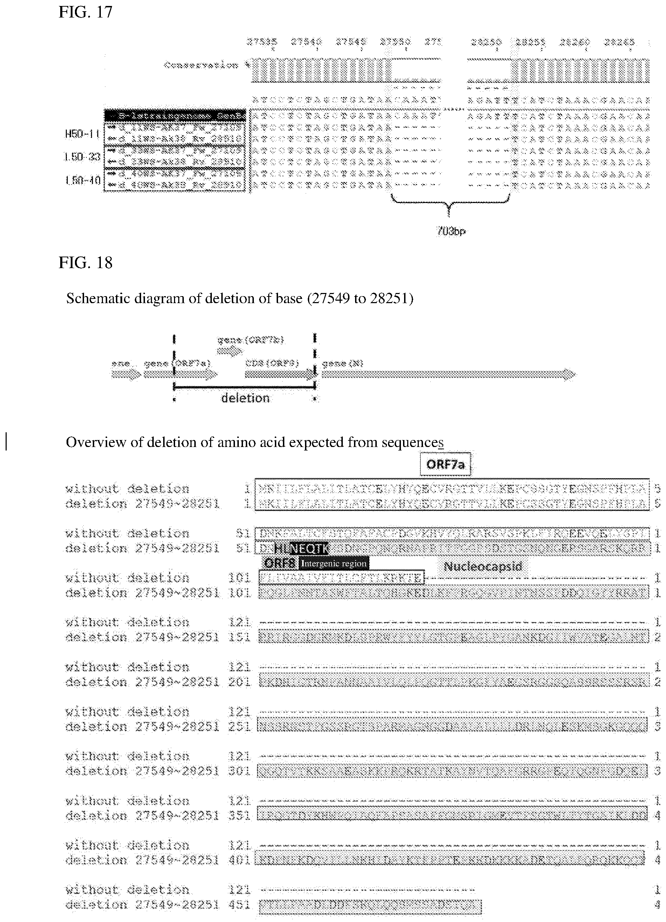

As a result, a deletion of a base sequence (SEQ ID NO: 7) of 703 bp at positions 27549 to 28251 as shown in Fig. 17 was found in all three strains. A schematic diagram of the deletion of the base sequence at positions 27549 to 28251 and a deletion of an amino acid sequence encoded thereby is shown in Fig. 18. In Fig. 18, ORF7a is a base sequence at positions 27394 to 27759, ORF7b is a base sequence at positions 27756 to 27887, and ORF8 is a base sequence at positions 27894 to 28259.

As shown in Fig. 18, the base sequence (SEQ ID NO: 7) region at positions 27549 to 28251 corresponds to a part (an amino acid sequence from position 53 to the terminal end. The same applies hereinafter.) of ORF7a, the entire ORF7b, and most of the amino acid sequence of ORF8. Since the deletion of this region involves a frameshift, it is considered that a protein is produced in which an amino acid sequence at positions 1 to 52 of ORF7a is fused with amino acid sequences encoded by eight bases at the 3' terminal, an intergenic region of ORF8 and a base sequence of a nucleocapsid. ORF7b is entirely deleted, and the original sequence of ORF8 is also entirely deleted.

### (2-2-4) Summary of mutations of temperature-sensitive strains (cold-adapted strains)

In the temperature-sensitive strains (cold-adapted strains), the H50-11 strain, the L50-33 strain, and the L50-40 strain, as shown in Table 3 below, mutations with check marks in the amino acid sequences of the SEQ ID NO indicated were found.

**[Table 3]**

| Mutation sign | Polypeptide | SEQ ID NO | Amino acid before mutation | Mutation position | Amino acid after mutation | H50-11 | L50-33 | L50-40 |
|---|---|---|---|---|---|---|---|---|
| (a') | NSP3 | 1 | V | 404 | A | ✔ | | |
| (b') | NSP3 | 1 | L | 445 | F | | ✔ | ✔ |
| (c') | NSP3 | 1 | K | 1792 | R | | ✔ | ✔ |
| (d') | NSP3 | 1 | D | 1832 | N | ✔ | | |
| (e') | NSP14 | 2 | G | 248 | V | | | |
| (f') | NSP14 | 2 | G | 416 | S | | | |
| (g') | NSP14 | 2 | A | 504 | V | | | |
| (h') | NSP16 | 3 | V | 67 | I | ✔ | | |
| (i) | Spike | 4 | L | 54 | W | | | ✔ |
| (j') | Spike | 4 | T | 739 | K | ✔ | | |
| (k') | Spike | 4 | A | 879 | V | | | |
| (l') | Envelope | 5 | L | 28 | P | | | |
| (m') | Nucleocapsid | 6 | S | 2 | F | | | |
| | ORF | Deletion of amino acid sequence encoded by base sequence of SEQ ID NO: 7 | | | | ✔ | ✔ | ✔ |

### [Test Example 3] Growth analysis of additional isolates, H50-11 strain, L50-33 strain, and L50-40 strain

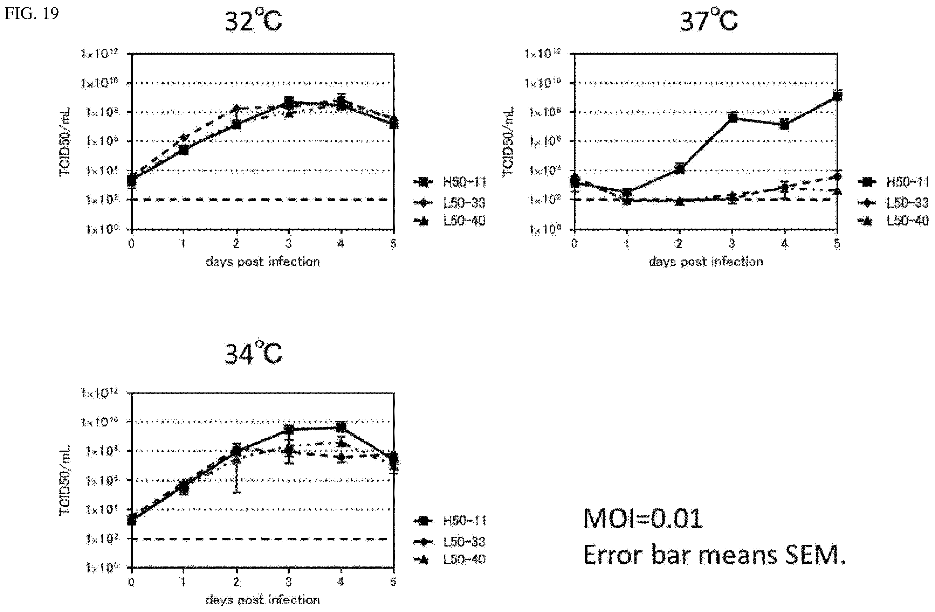

Vero cells were infected with additional isolates under conditions of MOI = 0.01 (N = 3). The viruses cultured at 37°C, 34°C, or 32°C, and each culture supernatant was collected at 0 to 5 d.p.i. The virus titer of theses culture supernatants were evaluated by TCID₅₀/mL using the Vero cells. The results are shown in Fig. 19. As a result, the additional isolates could growth at 32°C and 34°C, while showed delayed or decreased growth at 37°C.

### [Test Example 4] Pathogenicity analysis of each temperature-sensitive strain (4-1) Weight changes of hamsters infected with temperature-sensitive strains

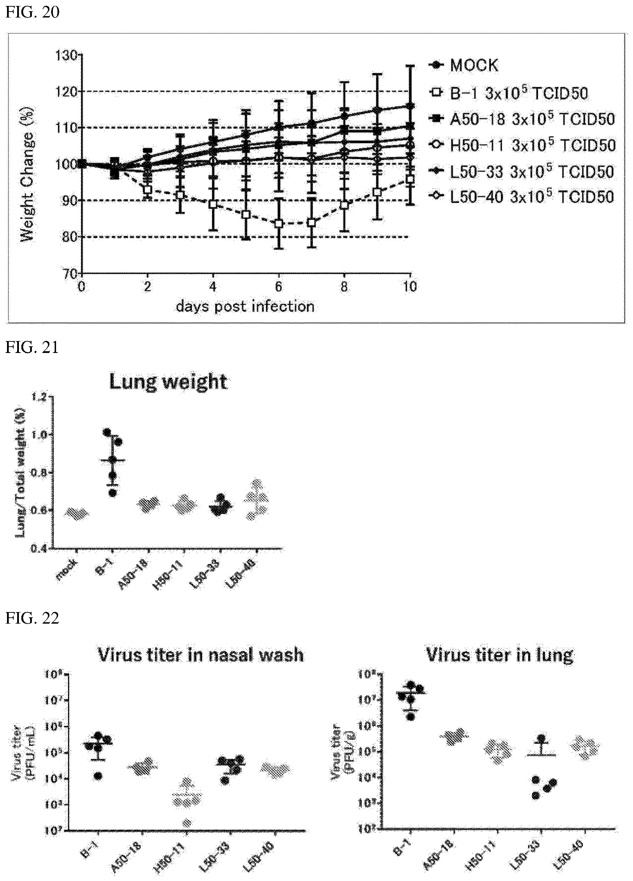

4-week-old male Syrian hamsters (n = 5) were bred for 1 week, and then the clinical isolate (B-1 strain) or the temperature-sensitive strain (A50-18 strain, L50-33 strain, L50-40 strain, or H50-11 strain) (3 × 10⁵ TCID50) was nasally administered in a volume of 100 µL, and weight changes for 10 days were observed. The group to which the same volume of D-MEM medium was nasally administered was defined as non-infected control (MOCK). The results were shown in Fig. 20. The B-1 strain-infected hamsters lose weight about 20% in 7 days, the hamsters infected with the temperature-sensitive strain did not lose weight significantly in all groups, suggesting that this strain showed significant lower pathogenicity than the wild type strain..

### (4-2) Evaluation of viral loads in the lungs and nasal wash specimens of hamsters infected with temperature-sensitive strains

4-week-old male Syrian hamsters (n = 5) were bred for 1 week, and then the clinical isolate (B-1 strain) or the temperature-sensitive strain (A50-18 strain, L50-33 strain, L50-40 strain, or H50-11 strain) (3 × 10⁵ TCID50) was nasally administered in a volume of 100 µL. Hamsters were euthanized at 3 dpi, and then nasal wash specimens were collected with 1 mL of D-PBS. In addition, the lungs of the hamsters were extracted, and the lung weight was measured, and then the right lungs were homogenized, and suspended with 1 mL of D-MEM, and then the supernatants were recovered as lung homogenates by centrifugation. The lung weight per total weight of the hamsters was shown in Fig. 21. In addition, the results of evaluating the viral loads in these nasal wash specimens and lung homogenates by plaque formation assay using Vero cells were shown in Fig. 22.

As a result of comparing lung weight per total weight of hamsters, the lung weight of the hamsters infected with the B-1 strain increased, and it was strongly suggested that the lungs swelled due to inflammation or the like. On the other hand, such increase in lung weight was not observed in the hamsters infected with the temperature-sensitive strains. In addition, as a result of comparing viral loads in the nasal wash specimens, there was no significant difference between the hamsters infected with the B-1 strain and the hamsters infected with the temperature-sensitive strains except the H50-11 strain, and the hamsters infected with the H50-11 strain had a small viral load in the nasal wash specimens. Furthermore, it was revealed that the viral loads in the lung homogenates of the hamsters infected with the temperature-sensitive strains were significantly less than those of the hamsters infected with the B-1 strain. From these results, it was suggested that each temperature-sensitive strain is an attenuated strain that cannot proliferate in a lower respiratory tract, similarly to the A50-18 strain in Test Example 1.

### [Test Example 5] Evaluation on immunogenicity of each temperature-sensitive strain (5-1) Wild-type strain challenge on hamsters infected with a temperature-sensitive strain

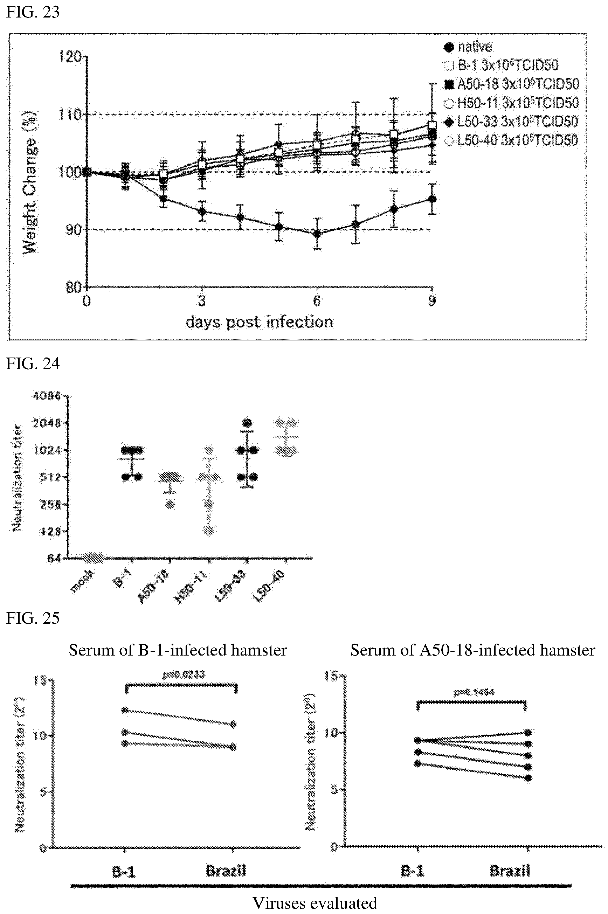

4-week-old male Syrian hamsters (n = 5) were bred for 1 week, and then the clinical isolate (B-1 strain) or the temperature-sensitive strain (A50-18 strain, L50-33 strain, L50-40 strain, or H50-11 strain) (3 × 10⁵ TCID50) was nasally administered in a volume of 100 µL. After 21 days, the clinical isolate (B-1 strain) (3 × 10⁵ TCID50) was nasally administered again in a volume of 100 µL, and weight changes for 9 days were observed. At this time, naive hamsters (n = 5) of the same age were used as a naive control. The results were shown in Fig. 23. The naive hamsters were lose weight due to infection with the B-1 strain, while the hamsters infected once with the B-1 strain and each temperature-sensitive strain did not lose weight. From this, it was revealed that immunity contributing to infection protection can be induced in infection not only by the B-1 strain, which is the wild strain, but also by each temperature-sensitive strain having low pathogenicity.

### (5-2) Neutralizing antibody induction evaluation of hamsters infected with temperature-sensitive strains

4-week-old male Syrian hamsters (n = 5) were bred for 1 week, and then the clinical isolate (B-1 strain) or the temperature-sensitive strain (A50-18 strain, L50-33 strain, L50-40 strain, or H50-11 strain) (3 × 10⁵ TCID50) was nasally administered in a volume of 100 µL. After 20 days, blood was collected partially and the obtained serum was used to evaluate neutralizing activity against the clinical isolate (B-1 strain). The neutralizing activity was measured in the same protocol as in (1-5 -2). The measurement results are shown in Fig. 24. It was revealed that antibodies which showed neutralizing activity were induced not only in the hamsters infected with the B-1 strain but also in the hamsters infected with each temperature-sensitive strain.

### [Test Example 6] Efficacy analysis on SARS-CoV-2 variant strain (6-1) Evaluation of neutralizing activity against SARS-CoV-2 variant strain of serum of hamsters infected with temperature-sensitive strain

4-week-old male Syrian hamsters (n = 3 or 5) were bred for 1 week, and then the clinical isolate (B-1 strain) or the temperature-sensitive strain (A50-18 strain) (3 × 10⁵ TCID50) was nasally administered in a volume of 100 µL. Partial blood collection was performed from hamsters at 3 weeks post infection, and the results of evaluation of neutralizing activity against the SARS-CoV-2 European type clinical isolate (B-1) and a Brazilian type variant (hCoV-19/Japan/TY7-503/2021 strain) using the obtained serum are shown in Fig. 25. The neutralizing activity was measured in the same protocol as in (1-5-2). It was revealed that the hamsters infected with the B-1 strain or the temperature-sensitive strain showed neutralizing activity against the Brazilian type variant. From this, it is considered that the present live attenuated vaccine may also be effective against SARS-CoV-2 variant.

### [Test Example 7] Comparative study experiments on administration routes and doses (7-1) Comparison of immunogenicity by administration routes

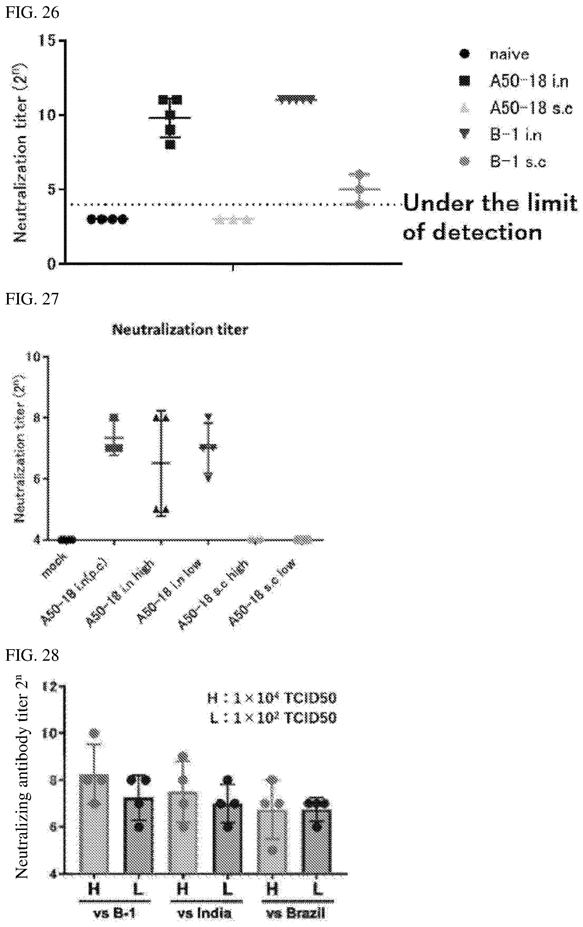

4-week-old male Syrian hamsters (n = 5) were bred for 1 week, and then the clinical isolate (B-1 strain) or the temperature-sensitive strain (A50-18 strain) (3 × 10⁵ TCID50) was nasally or subcutaneously administered in a volume of 100 µL. The untreated group was defined as a naive control (naive). After 3 weeks, the serum obtained by partial blood collection from the hamsters was used to evaluate neutralizing activity against a SARS-CoV-2 Brazilian type variant (hCoV-19/Japan/TY7-503/2021 strain). The neutralizing activity was measured in the same protocol as in (1-5-2). The results of the neutralizing activity were shown in Fig. 26. i.n denotes intranasal administration, and S.C denotes subcutaneous administration. By nasal administration of the B-1 strain or the A50-18 strain, neutralizing antibodies against authentic viruses could be induced. For subcutaneous administration, almost no neutralizing antibody could be induced at the tested dose, but in view of the results of nasal administration, it was considered that neutralizing antibodies can be induced even by subcutaneous administration when the dose is increased.

### (7-2) Comparison of immunogenicity by doses

4-week-old male Syrian hamsters (n = 5) were bred for 1 week, and then the temperature-sensitive strain (A50-18 strain) was administered nasally or subcutaneously. Doses are shown in Table 4.

**[Table 4]**

| | 1^{st} infection | |
|---|---|---|
| | Inoculation titer | Route |
| mock (n.c) | | |
| Robust titer infection (p.c) | 3.0 × 10⁵ TCID₅₀ | i.n 100 µL |
| High dose i.n | 1.0 × 10⁴ TCID₅₀ | i.n 10 µL |
| Low dose i.n | 1.0 × 10² TCID₅₀ | i.n 10 µL |
| High dose s.c | 1.0 × 10⁴ TCID₅₀ | s.c 100 µL |
| Low dose s.c | 1.0 × 10² TCID₅₀ | s.c 100 µL |

Partial blood collection was performed from hamsters at 3 weeks post infection, and the results of measuring neutralizing activity against the authentic viruses of a SARS-CoV-2 Brazilian type variant (hCoV-19/Japan/TY7-503/2021 strain) using the obtained serum are shown in Fig. 27. i.n denotes nasal administration, and S.C denotes subcutaneous administration. The neutralizing activity was measured in the same protocol as in (1-5 -2). Similarly to (7-1), by the nasal administration, an increase in neutralizing antibody titer was observed even in the low-dose administration group of 1 × 10² TCID50/10 µL. This suggested that the temperature-sensitive strain can induce sufficient immunity even by nasal administration of a small amount. For subcutaneous administration, almost no neutralizing antibody could be induced at the tested dose, but in view of the results of nasal administration, it was considered that neutralizing antibodies can be induced even by subcutaneous administration when the dose is increased.

### [Test Example 8] Evaluation of immunogenicity against SARS-CoV-2 variant strains

4-week-old male Syrian hamsters (n = 4) were bred for 1 week, and then the temperature-sensitive strain (A50-18 strain) of 1 × 10⁴ TCID50 or 1 × 10² TCID50 was nasally administered in a volume of 10 µL. Partial blood collection was performed from hamsters at 3 weeks after post infection, and the results of measuring neutralizing activity against authentic viruses of a SARS-CoV-2 European wild strain (B-1 strain), an Indian type variant (clinical isolate), and a Brazilian type variant (hCoV-19/Japan/TY7-503/2021 strain) using the obtained serum are shown in Fig. 28. The neutralizing activity was measured in the same protocol as in (1-5-2). It was revealed that even in an individual to which a small amount of the A50-18 strain was nasally administered, neutralizing antibodies against not only the B-1 strain, which is a parent strain and a wild-type strain, but also the Indian type variant strain and the Brazilian type variant strain could be induced dose-dependently .

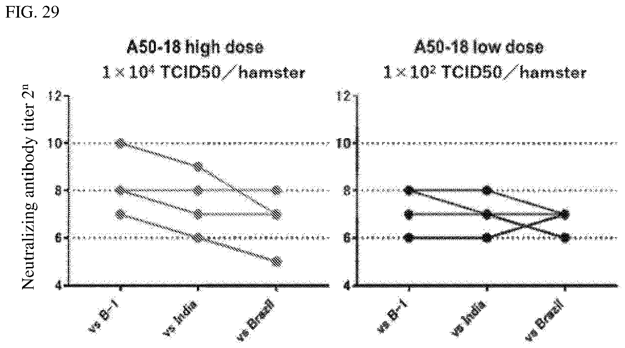

The results of comparing serum neutralizing antibody titers against each strain of serum of each individual are shown in Fig. 29. It was revealed that all individuals possessed neutralizing antibodies, although some individuals exhibited a low neutralizing antibody titer against the Brazilian type variant. From these results, it was suggested that immunity by nasal administration of the temperature-sensitive strains may exhibit cross-protection.

## Claims

1. A betacoronavirus cold-adapted strain comprising structural protein(s) and/or non-structural protein(s) having at least any one of the following mutations of (a) to (m):
(a) a mutation of an amino acid residue corresponding to valine at position 404 of an amino acid sequence set forth in SEQ ID NO: 1 in NSP3,
(b) a mutation of an amino acid residue corresponding to leucine at position 445 of an amino acid sequence set forth in SEQ ID NO: 1 in NSP3,
(c) a mutation of an amino acid residue corresponding to lysine at position 1792 of an amino acid sequence set forth in SEQ ID NO: 1 in NSP3,
(d) a mutation of an amino acid residue corresponding to aspartic acid at position 1832 of an amino acid sequence set forth in SEQ ID NO: 1 in NSP3,
(e) a mutation of an amino acid residue corresponding to glycine at position 248 of an amino acid sequence set forth in SEQ ID NO: 2 in NSP14,
(f) a mutation of an amino acid residue corresponding to glycine at position 416 of an amino acid sequence set forth in SEQ ID NO: 2 in NSP14,
(g) a mutation of an amino acid residue corresponding to alanine at position 504 of an amino acid sequence set forth in SEQ ID NO: 2 in NSP14,
(h) a mutation of an amino acid residue corresponding to valine at position 67 of an amino acid sequence set forth in SEQ ID NO: 3 in NSP16,
(i) a mutation of an amino acid residue corresponding to leucine at position 54 of an amino acid sequence set forth in SEQ ID NO: 4 in a spike,
(j) a mutation of an amino acid residue corresponding to threonine at position 739 of an amino acid sequence set forth in SEQ ID NO: 4 in a spike,
(k) a mutation of an amino acid residue corresponding to alanine at position 879 of an amino acid sequence set forth in SEQ ID NO: 4 in a spike,
(l) a mutation of an amino acid residue corresponding to leucine at position 28 of an amino acid sequence set forth in SEQ ID NO: 5 in an envelope, and
(m) a mutation of an amino acid residue corresponding to serine at position 2 of an amino acid sequence set forth in SEQ ID NO: 6 in a nucleocapsid.

2. The virus cold-adapted strain according to claim 1, wherein the betacoronavirus is SARS-CoV-2.

3. The virus cold-adapted strain according to claim 1 or 2, wherein a growth capability at a human lower respiratory tract temperature is decreased as compared with a growth capability of a betacoronavirus containing structural protein(s) and non-structural protein(s) not having any of the mutations of (a) to (m).

4. The virus cold-adapted strain according to claim 3, wherein the human lower respiratory tract temperature is 36 to 38°C.

5. The virus cold-adapted strain according to any one of claims 1 to 4, wherein the mutation of (a) is a substitution with alanine, the mutation of (b) is a substitution with phenylalanine, the mutation of (c) is a substitution with arginine, the mutation of (d) is a substitution with asparagine, the mutation of (e) is a substitution with valine, the mutation of (f) is a substitution with serine, the mutation of (g) is a substitution with valine, the mutation of (h) is a substitution with isoleucine, the mutation of (i) is a substitution with tryptophan, and/or the mutation of (j) is a substitution with lysine, the mutation of (k) is a substitution with valine, the mutation of (1) is a substitution with proline, and/or the mutation of (m) is a substitution with phenylalanine.

6. The virus cold-adapted strain according to any one of claims 1 to 5, comprising:
the NSP3 having the mutation of (a), the mutation of (b), the mutation of (c), and/or the mutation of (d) in the amino acid sequence set forth in SEQ ID NO: 1;
the NSP14 having the mutation of (e), the mutation of (f), and/or the mutation of (g) in the amino acid sequence set forth in SEQ ID NO: 2;
the NSP16 having the mutation of (h) in the amino acid sequence set forth in SEQ ID NO: 3;
the spike having the mutation of (i), the mutation of (j), and/or the mutation of (k) in the amino acid sequence set forth in SEQ ID NO: 4;
the envelope having the mutation of (1) in the amino acid sequence set forth in SEQ ID NO: 5; and/or
the nucleocapsid having the mutation of (m) in the amino acid sequence set forth in SEQ ID NO: 6.

7. The virus cold-adapted strain according to any one of claims 1 to 6, comprising the mutation of (e), the mutation of (f), the mutation of (g), the mutation of (k), the mutation of (1), and the mutation of (m).

8. The virus cold-adapted strain according to any one of claims 1 to 6, comprising the mutation of (a), the mutation of (d), the mutation of (h), and the mutation of (j).

9. The virus cold-adapted strain according to any one of claims 1 to 6, comprising the mutation of (b) and the mutation of (c).

10. The virus cold-adapted strain according to any one of claims 1 to 6, comprising the mutation of (b), the mutation of (c), and the mutation of (i).

11. The virus cold-adapted strain according to any one of claims 8 to 10, comprising a deletion of an amino acid sequence encoded by a base sequence set forth in SEQ ID NO: 7.

12. A live attenuated vaccine comprising the virus cold-adapted strain according to any one of claims 1 to 11.

13. The live attenuated vaccine according to claim 12, which is administered nasally.

14. The live attenuated vaccine according to claim 12, which is administered intramuscularly, subcutaneously, or intradermally.

15. A betacoronavirus gene vaccine comprising a gene encoding structural protein(s) and/or non-structural protein(s) having at least any one of the following mutations of (a) to (m):
(a) a mutation of an amino acid residue corresponding to valine at position 404 of an amino acid sequence set forth in SEQ ID NO: 1 in NSP3,
(b) a mutation of an amino acid residue corresponding to leucine at position 445 of an amino acid sequence set forth in SEQ ID NO: 1 in NSP3,
(c) a mutation of an amino acid residue corresponding to lysine at position 1792 of an amino acid sequence set forth in SEQ ID NO: 1 in NSP3,
(d) a mutation of an amino acid residue corresponding to aspartic acid at position 1832 of an amino acid sequence set forth in SEQ ID NO: 1 in NSP3,
(e) a mutation of an amino acid residue corresponding to glycine at position 248 of an amino acid sequence set forth in SEQ ID NO: 2 in NSP14,
(f) a mutation of an amino acid residue corresponding to glycine at position 416 of an amino acid sequence set forth in SEQ ID NO: 2 in NSP14,
(g) a mutation of an amino acid residue corresponding to alanine at position 504 of an amino acid sequence set forth in SEQ ID NO: 2 in NSP14,
(h) a mutation of an amino acid residue corresponding to valine at position 67 of an amino acid sequence set forth in SEQ ID NO: 3 in NSP16,
(i) a mutation of an amino acid residue corresponding to leucine at position 54 of an amino acid sequence set forth in SEQ ID NO: 4 in a spike,
(j) a mutation of an amino acid residue corresponding to threonine at position 739 of an amino acid sequence set forth in SEQ ID NO: 4 in a spike,
(k) a mutation of an amino acid residue corresponding to alanine at position 879 of an amino acid sequence set forth in SEQ ID NO: 4 in a spike,
(l) a mutation of an amino acid residue corresponding to leucine at position 28 of an amino acid sequence set forth in SEQ ID NO: 5 in an envelope, and
(m) a mutation of an amino acid residue corresponding to serine at position 2 of an amino acid sequence set forth in SEQ ID NO: 6 in a nucleocapsid.

16. The gene vaccine according to claim 15, which is administered nasally, intramuscularly, subcutaneously, or intradermally.
